(19) Europäisches Patentamt
European Patent Office
Office européen des brevets

(11) EP 0 955 289 B1

(12) EUROPÄISCHE PATENTSCHRIFT

(45) Veröffentlichungstag und Bekanntmachung des Hinweises auf die Patenterteilung:
**07.05.2003 Patentblatt 2003/19**

(51) Int Cl.[7]: **C07C 251/24**, C11D 3/395

(21) Anmeldenummer: **99810266.9**

(22) Anmeldetag: **29.03.1999**

(54) **Verfahren zur Behandlung von Textilmaterialien**

Method of treatment of textiles

Procédé de traitement de textiles

(84) Benannte Vertragsstaaten:
**AT BE CH DE DK ES FR GB GR IT LI NL PT SE**

(30) Priorität: **06.04.1998 EP 98810289**

(43) Veröffentlichungstag der Anmeldung:
**10.11.1999 Patentblatt 1999/45**

(73) Patentinhaber: **Ciba Specialty Chemicals Holding Inc.**
**4057 Basel (CH)**

(72) Erfinder:
- **Bachmann, Frank**
  **79106 Freiburg (DE)**
- **Dannacher, Josef**
  **4053 Basel (CH)**
- **Makowka, Cornelia**
  **79725 Laufenburg (DE)**
- **Schlingloff, Gunther**
  **4125 Riehen (CH)**
- **Weingartner, Peter**
  **4457 Diegten (CH)**
- **Richter, Grit**
  **79395 Neuenburg (DE)**

(56) Entgegenhaltungen:
**EP-A- 0 587 552**
**EP-A- 0 630 964**
**EP-A- 0 693 550**
**EP-A- 0 717 103**
**EP-A- 0 902 083**
**WO-A-91/14694**
**WO-A-95/21172**
**WO-A-96/40148**
**WO-A-97/19162**
**DE-A- 19 529 905**

Bemerkungen:
Die Akte enthält technische Angaben, die nach dem Eingang der Anmeldung eingereicht wurden und die nicht in dieser Patentschrift enthalten sind.

EP 0 955 289 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft ein Verfahren zur Verhinderung des Wiederaufziehens von migrierenden Farbstoffen in Waschflotten, enthaltend ein Peroxid und einen Mangankatalysator, für das Verfahren geeignete Mangankatalysatoren sowie Waschmittelzubereitungen, enthaltend, diese Katalysatoren.

Es ist bereits bekannt, dass manche Mangankomplexe vom Salentyp geeignete Katalysatoren für Oxidationen mit Persauerstoffverbindungen, insbesondere im Rahmen eines Waschprozesses sind. Es handelt sich dabei ausschliesslich um symmetrische Salenkomplexe. Ausserdem ist schon beschrieben, dass gewisse andere Mangankomplexe ausgeprägte Bleichwirkung auf Schmutz und Farbstoffe in Waschflotten besitzen.

Aus DE 195 29 905 A ist bekannt, dass symmetrische Mangankomplexe als Bleichkatalysatoren eingesetzt werden können.

Aus WO 91 14694 A und WO 96 40148 sind symmetrische Mangankomplexe bekannt, die als Katalysatoren zur Epoxidierung von prochiralen Olefinen eingesetzt werden.

Aus WO 95 21172 A sind Übergangsmetallkomplexe bekannt, die als Antioxidantien zur Bekämpfung von Krankheiten eingesetzt werden, sowie ein Verfahren zur Herstellung unsymmetrischer Liganden durch Umsetzung von geschützten Diaminen mit zwei unterschiedlich substituierten Salicylaldeydresten oder Salicylketonresten.

Es wurde nun gefunden, dass bestimmte unsymmetrische Mangankomplexe vom Salen-Typ eine deutlich stärkere spezifische Wirkung als Katalysatoren zur Verhinderung des Wiederaufziehens von migrierenden Farbstoffen in Waschflotten zeigen, ohne zu nennenswerten Farbstoff - und Faserschädigungen Anlass zu geben. Als unsymmetrisch werden Komplexe bezeichnet, die durch den Umsatz von 2 mol zweier verschieden modifizierter Salicyaldehyde bzw. o-Hydroxyphenylketone mit 1 mol Ethylendiamin oder eines anderen modifizierten Diaminbausteines entstehen und somit zwei verschieden substituierte aromatische Reste besitzen.

Die vorliegende Erfindung betrifft somit ein Verfahren zur Verhinderung des Wiederaufziehens von migrierenden Farbstoffen in einer Waschflotte, das dadurch gekennzeichnet ist, dass man der Waschflotte, die ein peroxidhaltiges Waschmittel enthält, 0,5 bis 150, vorzugsweise 1,5 bis 75, insbesondere 7,5 bis 40 mg, pro Liter Waschflotte einer oder mehrerer Verbindungen der Formel

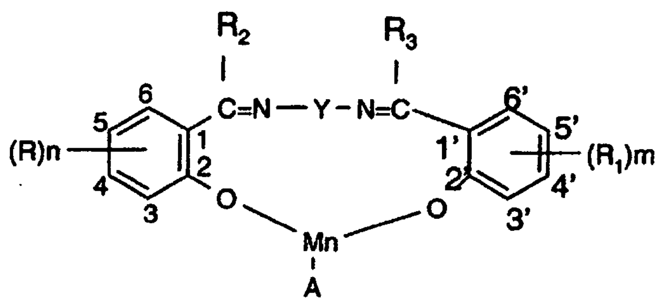

(1)

zusetzt, worin

- n 0, 1 , 2 oder 3,
- m 1, 2 oder 3,
- A ein Anion;
- Y ein linearer oder verzweigter Alkylenrest der Formel $-[C(R_5)_2]_r-$, wobei r eine ganze Zahl von 1 bis 8 und die $R_5$-Reste unabhängig voneinander Wasserstoff oder $C_1$-$C_4$-alkyl bedeuten;
  -CX=CX-, worin X Cyano, lineares oder verzweigtes $C_1$-$C_8$-alkyl oder Di(lineares oder verzweigtes $C_1$-$C_8$-alkyl)-amino,
  $-(CH_2)_q-NR_4-(CH_2)_q-$, worin $R_4$ Wasserstoff oder lineares oder verzweigtes $C_1$-$C_4$-alkyl und
  q 1, 2, 3 oder 4; oder
  ein 1,2-Cyclohexylenrest der Formel:

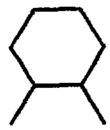

oder

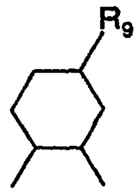

oder ein 1,2-Arylrest der Formel

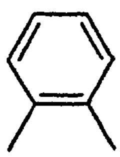

oder

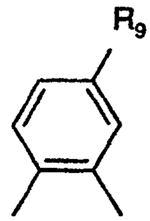

worin $R_9$ $SO_3H$, $CH_2OH$ oder $CH_2NH_2$ ist,
R und $R_1$ unabhängig voneinander Cyano, Halogen, $OR_5$ oder $COOR_5$, worin $R_5$ Wasserstoff oder lineares oder verzweigtes $C_1$-$C_4$-alkyl ist, Nitro, lineares oder verzweigtes $C_1$-$C_8$-alkyl, lineares oder verzweigtes teilfluoriertes oder perfluoriertes $C_1$-$C_8$-alkyl, $NHR_6$ oder $NR_6R_7$, worin $R_6$ und $R_7$ gleich oder verschieden sind und je lineares oder verzweigtes $C_1$-$C_{12}$-alkyl bedeuten oder worin $R_6$ und $R_7$ zusammen mit dem sie verbindenden N-Atom einen 5-, 6-oder 7-gliedrigen Ring bilden, der weitere Heteroatome enthalten kann, oder lineares oder verzweigtes $C_1$-$C_8$-alkyl-$R_8$, worin $R_8$ ein Rest $OR_5$, $COOR_5$ oder $NR_6R_7$ mit den vorstehend genannten Bedeutungen oder $NH_2$ bedeutet, oder $-N^{\oplus}R_4R_6R_7$, worin $R_4$, $R_6$ und $R_7$ die vorstehend genannten Bedeutungen haben,
$R_2$ und $R_3$ unabhängig voneinander Wasserstoff, lineares oder verzweigtes $C_1$-$C_4$ alkyl oder unsubstituiertes Aryl oder Aryl, das durch Cyano, Halogen, $OR_5$ oder $COOR_5$ worin $R_5$ Wasserstoff oder lineares oder verzweigtes $C_1$-$C_4$-alkyl ist, Nitro, lineares oder verzweigtes $C_1$-$C_8$-alkyl, $NHR_6$ oder $NR_6R_7$, worin $R_6$ und $R_7$ gleich oder verschieden sind und die vorstehend angegebenen Bedeutungen haben, oder lineares oder verzweigtes $C_1$-$C_8$-alkyl-$R_8$, worin $R_8$ ein Rest $OR_5$,$COOR_5$ oder $NR_6R_7$ mit den vorstehend genannten Bedeungen oder $NH_2$ bedeutet, oder $-N^{\oplus}R_4R_6R_7$, worin $R_4$, $R_6$ und $R_7$ die vorstehend genannten Bedeutungen haben, substituiert ist,
mit der Bedingung, dass R und $R_1$ nicht die gleiche Bedeutung haben, falls n und m identisch sind.

Bei den Verbindungen der Formel (1), bei denen n 2 oder 3 bedeutet, können die Reste R die gleiche oder unterschiedliche Bedeutungen aufweisen. Das gleiche gilt für Verbindungen der Formel (1), bei denen m 2 oder 3 bedeutet, bezüglich der Reste $R_1$.

Bedeutet Y einen 1,2-Cydohexylen-Rest, so kann dieser in jeder seiner stereoisomeren cis/trans Formen vorliegen.

Vorzugsweise bedeutet Y einen Rest der Formel $-(CH_2)_r-$, wobei r eine ganze Zahl von 1 bis 8 bedeutet oder der Formel $-C(R_5)_2-(CH_2)_p-C(R_5)_2-$ worin p eine Zahl von 0 bis 6 und $R_5$ Wasserstoff oder $C_1$-$C_4$-alkyl bedeutet.

In besonders bevorzugten Verbindungen der Formel (1) ist Y ein Rest der Formel $-(CH_2)_r-$, wobei r eine ganze Zahl von 1 bis 4 bedeutet oder der Formel $-(CR_5)_2-(CR_5)_2-$, worin $R_5$ unabhängig voneinander Wasserstoff oder Methyl bedeutet.

Halogen bedeutet vorzugsweise Chlor, Brom oder Fluor, wobei Chlor besonders bevorzugt ist

Falls n oder m 1 bedeuten, befinden sich die Gruppen R und $R_1$ vorzugsweise in 4-Stellung des jeweiligen Benzolrings, ausser, wenn R bzw. $R_1$ Nitro oder $COOR_5$ bedeutet. In diesem Falle ist die Gruppe R bzw. $R_1$ vorzugsweise in 5-Stellung.

Falls n oder m 2 bedeuten, befinden sich die beiden R-Gruppen oder $R_1$-Gruppen vorzugsweise in 4,6-Stellung des jeweiligen Benzolrings, ausser, wenn R bzw. $R_1$ Nitro oder $COOR_5$ bedeutet In diesem Falle sind die beiden R-Gruppen bzw. $R_1$-Gruppen vorzugsweise in 3,5-Stellung.

Falls R oder $R_1$ Di-($C_1$-$C_{12}$alkyl)amino bedeutet, so kann die Alkylgruppe geradkettig oder verzweigt sein. Vorzugsweise enthält sie 1 bis 8, insbesondere 1 bis 4 und vor allem 1 oder 2 Kohlenstoffatome.

Vorzugsweise bedeuten die Reste R und $R_1$ Wasserstoff, Nitro, $OR_5$, $COOR_5$ oder $N(R_5)_2$, wobei $R_5$ Wasserstoff oder $C_1$-$C_4$-alkyl, vor allem Methyl oder Ethyl ist.

Die Reste $R_2$ und $R_3$ bedeuten vor allem Wasserstoff, Methyl, Ethyl oder unsubstituiertes Phenyl.

Aryl bedeutet z. B. Naphthyl oder vor allem Phenyl.

Falls $R_6$ und $R_7$ zusammen mit dem sie verbindenden N-Atom einen 5-, 6- oder 7-Ring bilden, so handelt es sich vor allem um einen Pyrrolidin-, Piperidin-, Morpholin- oder Piperazinring. Der Piperazinring kann am nicht mit dem Phenylrest verbundenen N-Atom substituiert sein, z. B. durch Alkyl.

Geeignete Anionen sind beispielsweise Halogenid wie z.B. Chlorid, Perchlorat, Sulfat, Nitrat, Hydroxid, $BF_4^-$, $PF_6^-$, Carboxylat, Acetat; Tosylat oder Triflat. Bevorzugt unter diesen sind Chlorid, Acetat und Carboxylat.

Einen weiteren Gegenstand der vorliegenden Erfindung stellen die Verbindungen der Formel

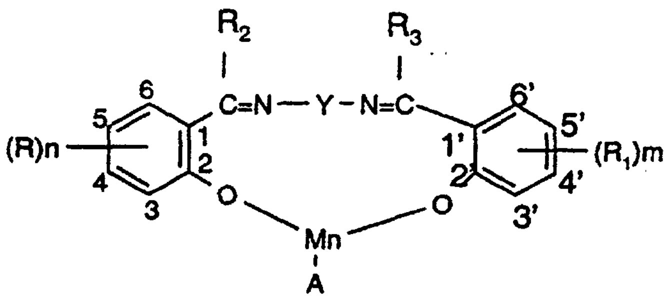

(1a)

dar, worin

n 0, 1, 2 oder 3,
m 1, 2 oder 3,
A ein Anion;
Y ein linearer oder verzweigter Alkylenrest der Formel $-[C(R_5)_2]_r-$, wobei r eine ganze Zahl von 1 bis 8 und die $R_5$-Reste unabhängig voneinander Wasserstoff oder $C_1$-$C_4$-alkyl bedeuten;
-CX=CX-, worin X Cyano, lineares oder verzweigtes $C_1$-$C_8$-alkyl oder Di(lineares oder verzweigtes $C_1$-$C_8$-alkyl)-amino,
$-(CH_2)_q-NR_4-(CH_2)_q-$, worin $R_4$ die vorstehend genannte Bedeutung hat und q 1, 2, 3 oder 4; oder
ein 1,2-Cyclohexylenrest der Formel:

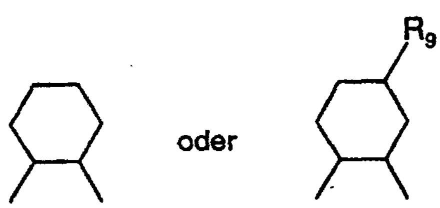

oder ein 1,2-Arylrest der Formel

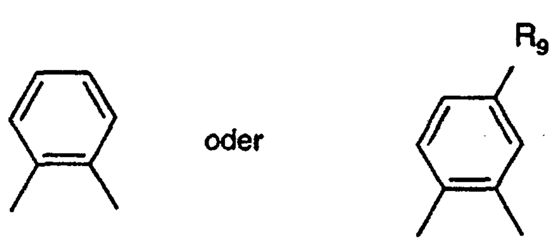

worin $R_9$ $SO_3H$, $CH_2OH$ oder $CH_2NH_2$ ist,
R und $R_1$ unabhängig voneinander Cyano, Halogen, $OR_5$ oder $COOR_5$, worin $R_5$ Wasserstoff oder lineares oder verzweigtes $C_1$-$C_4$-alkyl ist, Nitro, lineares oder verzweigtes $C_1$-$C_8$-alkyl, lineares oder verzweigtes teilfluoriertes oder perfluoriertes $C_1$-$C_8$-alkyl, $NHR_6$ oder $NR_6R_7$, worin $R_6$ und $R_7$ gleich oder verschieden sind und je lineares oder ver-

zweigtes $C_1$-$C_{12}$-alkyl bedeuten oder worin $R_6$ und $R_7$ zusammen mit dem sie verbindenden N-Atom einen 5-, 6-oder 7-gliedrigen Ring bilden, der weitere Heteroatome enthalten kann, oder lineares oder verzweigtes $C_1$-$C_8$-alkyl-$R_8$, worin $R_8$ ein Rest $OR_5$,$COOR_5$ oder $NR_6R_7$ mit den vorstehend genannten Bedeungen oder $NH_2$ bedeutet, oder -$N^{\oplus}R_4R_6R_7$, worin $R_4$, $R_6$ und $R_7$ die vorstehend genannten Bedeutungen haben,

$R_2$ und $R_3$ unabhängig voneinander Wasserstoff, lineares oder verzweigtes $C_1$-$C_4$ alkyl oder unsubstituiertes Aryl oder Aryl, das durch Cyano, Halogen, $OR_5$ oder $COOR_5$, worin $R_5$ Wasserstoff oder lineares oder verzweigtes $C_1$-$C_4$-alkyl ist, Nitro, lineares oder verzweigtes $C_1$-$C_8$-alkyl, $NHR_6$ oder $NR_6R_7$, worin $R_6$ und $R_7$ gleich oder verschieden sind und je lineares oder verzweigtes $C_1$-$C_{12}$-alkyl bedeuten oder worin $R_6$ und $R_7$ zusammen mit dem sie verbindenden N-Atom einen 5-, 6- oder 7-gliedrigen Ring bilden, der weitere Heteroatome enthalten kann, oder lineares oder verzweigtes $C_1$-$C_8$-alkyl-$R_8$, worin $R_8$ ein Rest

$OR_5$, $COOR_5$ oder $NR_6R_7$ mit den vorstehend genannten Bedeutungen oder $NH_2$ bedeutet, oder -$N^{\oplus}R_4R_6R_7$, worin $R_4$, $R_6$ und $R_7$ die vorstehend genannten Bedeutungen haben, substituiert ist,

mit der Bedingung, dass R und $R_1$ nicht die gleiche Bedeutung haben, falls n und m identisch sind und $R_2$ und $R_3$ beide Wasserstoff bedeuten und dass von den Resten $R_2$ und $R_3$ nicht der eine Wasserstoff und der andere Phenyl bedeutet.

Die vorstehend bei den Mangankomplexen der Formel (1) angegebenen bevorzugten Bedeutungen für n, m, Y, A, R, $R_1$, $R_2$ und $R_3$ sind auch für die Verbindungen der Formel (1a) bevorzugt.

Die Verbindungen der Formel (1) und (1a) werden z. B. auf an sich bekannte Art und Weise aus den entsprechenden Liganden und einer Manganverbindung hergestellt. Solche Herstellungsverfahren sind beispielsweise in den US-Patenten 5,281,578 und 4,066,459 beschrieben. Alle dort erwähnten Mangankomplexe haben jedoch symmetrisch substituierte Liganden. Überraschenderweise zeichnen sich die erfindungsgemässen Mangankomplexe mit unsymmetrischen Liganden durch verbesserte spezifische Wirkung als Katalysatoren für die Oxidationen mit Peroxiden aus.

Die Liganden der Formel

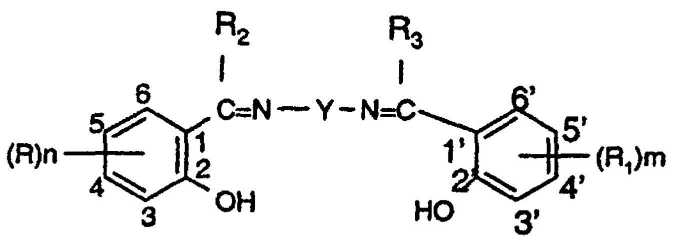

(2)

worin R, $R_1$, $R_2$, $R_3$, Y, n und m die unter der Formel (1a) angegebene Bedeutung haben, sind ebenfalls neu. Sie werden auf an sich bekannte Art und Weise hergestellt, beispielsweise, indem man ein Diamin der Formel $H_2N$-Y-$NH_2$ zunächst mit einem Aldehyd oder Keton der Formel

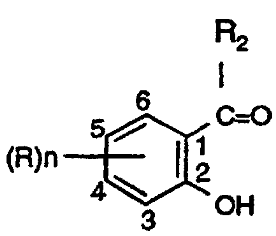

(3)

und danach mit einem Aldehyd oder Keton der Formel

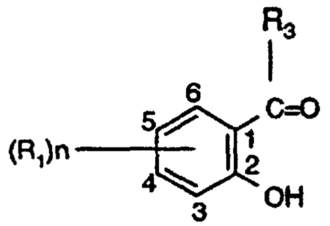

(4)

umsetzt. In den Formeln (3) und (4) weisen R, $R_1$, $R_2$, $R_3$, n und m die unter der Formel (1) angegebenen Bedeutungen auf, mit der Bedingung, dass R und $R_1$ nicht die gleiche Bedeutung aufweisen, wenn n und m identisch sind.

Von besonderem Interesse für die Verwendung im erfindungsgemässen Verfahren sind die Verbindungen der Formel

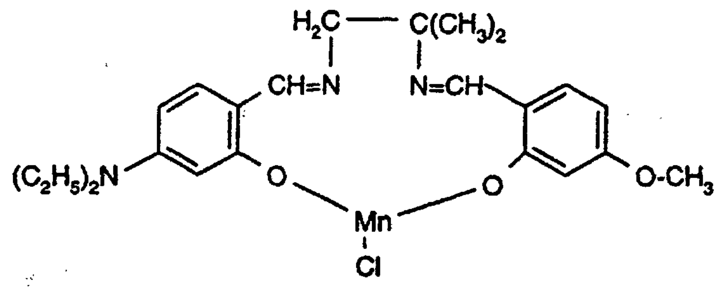

(1A)

und

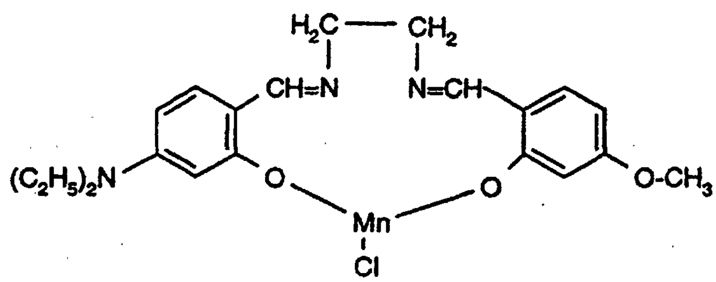

(1B)

Es ist auch möglich, die Verbindungen der Formel (1) zusammen mit entsprechenden analogen symmetrischen Mangankomplexen einzusetzen, d. h. mit Verbindungen der Formel (1), bei denen $(R)_n$ und $(R_1)_m$ identisch sind. Solche Mischungen erhält man z. B. dadurch, dass man bei der vorstehenden Synthese der Uganden der Formel (2) ein Diamin der Formel $H_2N\text{-}Y\text{-}NH_2$ mit einer Mischung aus zwei verschiedenen Verbindungen der Formel (3) umsetzt und das erhaltene Gemisch, enthaltend einen unsymmetrischen und zwei symmetrische Liganden der Formel (2), in die entsprechenden Mn-Komplexe umwandelt.

Die vorliegende Erfindung betrifft ausserdem ein Waschmittel, enthaltend

I) 5 - 90 %, vorzugsweise 5 - 70 % A) eines anionischen Tensids und/oder B) eines nichtionischen Tensids,
II) 5 - 70 %, vorzugsweise 5 - 50 %, insbesondere 5 - 40 % C) einer Buildersubstanz,
III) 0,1 - 30 %, vorzugsweise 1 - 12 % D) eines Peroxids und
IV) 0,005 - 2 %, vorzugsweise 0,02 -1 %, insbesondere 0,1 - 0,5 % E) einer Verbindung der vorstehend definierten Formel (1), wobei die Prozentangaben jeweils Gewichtsprozente, bezogen auf das Gesamtgewicht des Waschmittels bedeuten.

Das Waschmittel kann in fester oder flüssiger Form vorliegen, beispielsweise als flüssiges, nichtwässriges Waschmittel, enthaltend nicht mehr als 5, vorzugsweise 0 bis 1 Gew. % Wasser, und als Basis eine Suspension einer Buildersubstanz in einem nichtionischen Tensid haben, z. B. wie in der GB-A-2,158,454 beschrieben.

Vorzugsweise liegt das Waschmittel jedoch als Pulver oder Granulat vor.

Dieses kann z. B. hergestellt werden, indem man zunächst ein Ausgangspulver herstellt durch Sprühtrocknen einer wässrigen Anschlämmung, enthaltend alle vorstehend aufgeführten Komponenten ausser den Komponenten D) und E), und anschliessend die trockenen Komponenten D) und E) zugibt und alles miteinander vermischt.

Man kann auch die Komponente E) zu einer wässrigen Anschlämmung, enthaltend die Komponenten A), B) und C), zugeben, danach sprühtrocknen und dann die Komponente D) mit der trockenen Masse vermischen.

Es ist ausserdem möglich, von einer wässrigen Anschlämmung auszugehen, die zwar die Komponenten A) und C), die Komponente B) aber nicht oder nur teilweise enthält. Die Anschlämmung wird sprühgetrocknet, dann die Komponente E) mit der Komponente B) vermischt und zugesetzt und anschliessend wird die Komponente D) trocken zugemischt.

Das anionische Tensid A) kann z. B. ein Sulfat-, Sulfonat- oder Carboxylat-Tensid oder eine Mischung aus diesen sein.

Bevorzugte Sulfate sind solche mit 12 - 22 C-Atomen im Alkylrest, ggf. in Kombination mit Alkylethoxysulfaten, deren Alkylrest 10 - 20 C-Atome besitzt.

Bevorzugte Sulfonate sind z. B. Alkylbenzolsulfonate mit 9 - 15 C-Atomen im Alkylrest.

Das Kation bei den anionischen Tensiden ist vorzugsweise ein Alkalimetallkation, insbesondere Natrium.

Bevorzugte Carboxylate sind Alkalimetallsarcosinate der Formel $R\text{-}CO\text{-}N(R^1)\text{-}CH_2COOM^1$, worin R Alkyl oder Alkenyl mit 8-18 C-Atomen im Alkyl- oder Alkenylrest, $R^1$ $C_1$-$C_4$-alkyl und $M^1$ ein Alkalimetall bedeutet.

Das nichtionische Tensid B) kann z. B. ein Kondensationsprodukt von 3 - 8 Mol Ethylenoxid mit 1 Mol primärem Alkohol, der 9 - 15 C-Atome besitzt, sein.

Als Buildersubstanz C) kommen z. B. Alkalimetallphosphate, insbesondere Tripolyphosphate, Karbonate oder Bikarbonate, insbesondere deren Natriumsalze, Silikate, Aluminiumsilikate, Polycarboxylate, Polycarbonsäuren, organische Phosphonate, Aminoalkylenpoly(alkylenphosphonate) oder Mischungen dieser Verbindungen in Betracht.

Besonders geeignete Silikate sind Natriumsalze von kristallinen Schichtsilikaten der Formel $NaHSi_tO_{2t+1}.pH_2O$ oder $Na_2Si_tO_{2t+1}.pH_2O$, worin t eine Zahl zwischen 1,9 und 4 und p eine Zahl zwischen 0 und 20 ist.

Von den Aluminiumsilikaten sind die kommerziell unter den Namen Zeolith A, B, X und HS erhältlichen bevorzugt sowie Mischungen, enthaltend zwei oder mehrere dieser Komponenten.

Bevorzugt unter den Polycarboxylaten sind die Polyhydroxycarboxylate, insbesondere Citrate, und Acrylate sowie deren Copolymere mit Maleinsäureanhydrid.

Bevorzugte Polycarbonsäuren sind Nitrilotriessigsäure, Ethylendiamintetraessigsäure sowie Ethylendiamindisuccinat sowohl in racemischer Form als auch die enantiomerenreine S,S-Form.

Besonders geeignete Phosphonate oder Aminoalkylenpoly(alkylenphosphonate) sind Alkalimetallsalze der 1-Hydroxyethan-1,1-diphosphonsäure, Nitrilotris(methylenphosphonsäure), Ethylendiamintetramethylenphosphonsäure und Diethylentriaminpentamethylenphosphonsäure.

Als Peroxidkomponente D) kommen z. B. die in der Literatur bekannten und im Markt erhältlichen organischen und anorganischen Peroxide in Frage, die Textilmaterialien bei üblichen Waschtemperaturen, beispielsweise bei 10 bis 95 °C bleichen.

Bei den organischen Peroxiden handelt es sich beispielsweise um Mono- oder Polyperoxide, insbesondere um organische Persäuren oder deren Salze, wie Phthalimidoperoxycapronsäure, Peroxybenzoesäure, Diperoxydodecandisäure, Diperoxynonandisäure, Diperoxydecandisäure, Diperoxyphthalsäure oder deren Salze.

Vorzugsweise verwendet man jedoch anorganische Peroxide, wie z. B. Persulfate, Perborate, Percarbonate oder Persilikate. Man kann selbstverständlich auch Mischungen aus anorganischen und/oder organischen Peroxiden verwenden. Die Peroxide können in unterschiedlichen Kristallformen und mit unterschiedlichem Wassergehalt vorliegen und sie können auch zusammen mit anderen anorganischen oder organischen Verbindungen eingesetzt werden, um ihre Lagerstabilität zu verbessem.

Die Zugabe der Peroxide zu dem Waschmittel erfolgt vorzugsweise durch Mischen der Komponenten, z. B. mit Hilfe eines Schneckendosiersystems und/oder eines Fliessbettmischers.

Die Waschmittel können zusätzlich zu der erfindungsgemässen Kombination einen oder mehrere optische Aufheller enthalten, beispielsweise aus der Klasse Bis-triazinylaminostilben-disulfonsäure, Bis-triazolyl-stilben-disulfonsäure, Bis-styryl-biphenyl oder Bis-benzofuranylbiphenyl, ein Bis-benzoxalylderivat, Bis-benzimidazolylderivat, Cumarinderivat oder ein Pyrazolinderivat.

Femer können die Waschmittel Suspendiermittel für Schmutz, z. B. Natriumcarboxymethylcellulose, pH-Regulatoren, z. B.Alkali oder Erdalkalimetallsilikate, Schaumregulatoren, z. B. Seife, Salze zur Regelung der Sprühtrocknung und der Granuliereigenschaften, z. B. Natriumsulfat, Duftstoffe sowie gegebenenfalls, Antistatica und Weichspüler, Enzyme, wie Amylase, Bleichmittel, Pigmente und/oder Nuanciermittel enthalten. Diese Bestandteile müssen

selbstverständlich stabil gegenüber dem eingesetzten Bleichmittel sein.

Weitere bevorzugte Zusätze zu den erfindungsgemässen Waschmitteln sind Polymere, die Anschmutzungen beim Waschen von Textilien durch in der Waschflotte befindliche Farbstoffe, die sich unter Waschbedingungen von den Textilien abgelöst haben, verhindern. Vorzugsweise handelt es sich um Polyvinylpyrrolidone, die gegebenenfalls durch Einbau von anionischen oder kationischen Substituenten modifiziert sind, insbesondere um solche mit einem Molekulargewicht im Bereich von 5000 bis 60000, vor allem von 10000 bis 50000. Diese Polymere werden vorzugsweise in einer Menge von 0,05 bis 5 Gew. %, vor allem 0,2 bis 1,7 Gew. %, bezogen auf das Gesamtgewicht des Waschmittels, eingesetzt.

Zusätzlich können die erfindungsgemässen Waschmittel noch sog. Perborat-Aktivatoren, wie z.B. TAED oder TAGU enthalten. Bevorzugt ist TAED, das vorzugsweise in in einer Menge von 0,05 bis 5 Gew. %, vor allem 0,2 bis 1,7 Gew. %, bezogen auf das Gesamtgewicht des Waschmittels, eingesetzt wird.

Die folgenden Beispiele dienen zur Erläuterung der Erfindung, ohne sie darauf zu beschränken. Teile und Prozentangaben beziehen sich auf das Gewicht, falls nicht anders angegeben.

Beispiel 1:

N-Mono[4-(diethylamino)salicyliden]-2-methylpropan-1,2-diamin

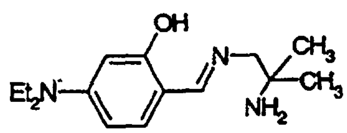

Man legt eine Lösung von 4.56 g (0.0517 mol) 1,2-Diamino-2-methylpropan in 50 ml Ethanol vor. Unter Rühren wird bei Raumtemperatur eine Lösung von 10.0 g (0.0517 mol) 4-N-Diethylaminosalicylaldehyd in 50 ml Ethanol innerhalb von 2 h hinzugetropft. Nach 2 h Rühren (DC-Kontrolle Acetonitril/Wasser 9:1) ist die Reaktion vollständig. Die Reaktionslösung wird vorsichtig eingeengt und im Hochvakuum getrocknet. Man erhält als Rohprodukt 13.6 g eines dunkelroten Öls, das ohne weitere Reinigung weiter eingesetzt wird.

Beispiel 2:

N-Mono[4-(dimethylamino)salicyliden]-2-methylpropan-1,2-diamin

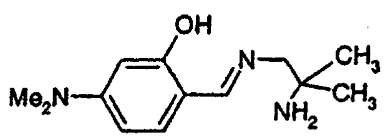

Zu 6.4 ml (5.3 g, 60.5 mmol) 1,2-Diamino-2-methylpropan wird unter Rühren bei Raumtemperatur eine Lösung von 10.0 g (60.53 mmol) 4-Dimethylaminosalicylaldehyd in 100 ml Ethanol innerhalb von 2 h hinzugetropft. Nach 2 h Rühren (DC-Kontrolle Acetonitril/Wasser 9:1) bei Raumtemperatur ist die Reaktion vollständig. Die Reaktionslösung wird vorsichtig eingeengt und im Hochvakuum getrocknet. Man erhält als Rohprodukt 14 g eines dunkelroten Öls, das ohne weitere Reinigung weiter umgesetzt wird.

Beispiel 3:

N-1-[4-(Diethylamino)salicyliden]-N'-2-(4-methoxysalicyliden)-2-methylpropan-1,2-diamin (Struktur I) und N-2-[4-(Diethylamino)salicyliden]-N'-1-(4-methoxysalicyliden)-2-methylpropan-1,2-diamin (Struktur II)

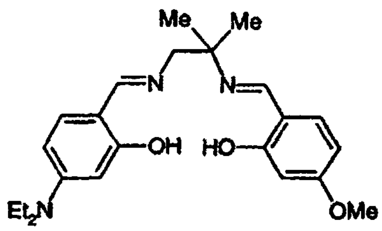

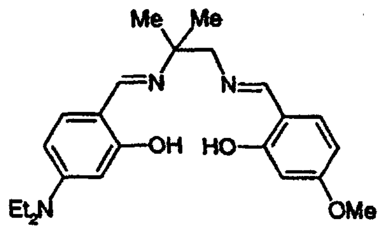

I

II

Eine Suspension von 13.6 g (0.0517 mol) N-Mono[4-(diethylamino)salicyliden]-2-methylpropan-1,2-diamin aus Beispiel 1 in 50 ml Ethanol erwärmt man unter Rühren auf 50 °C, bis man eine klare Lösung erhält. Man gibt 7.87 g (0.0517 mol) 4-Methoxysalicylaldehyd (fest) dazu und erwärmt 2 h auf Rückfluss. Danach ist der Umsatz vollständig (DC-Kontrolle Essigester/Methanol 9:1). Die Reaktionslösung wird danach vorsichtig eingeengt und im Hochvakuum getrocknet. Man erhält als Rohprodukt 20 g eines dunkelroten Öls, das langsam erstarrt. Die Reinigung erfolgt säulenchromatographisch (Laufmittelgemisch Essigester/Methanol 9:1). Das unsymmetrisch substituierte Salenderivat wird als Diastereomerenmischung (Struktur I und II) isoliert. Ausbeute: 7 g, rötliches Öl (34 %). Die Charakterisierung des Produktes erfolgt durch $^1$H und $^{13}$C-NMR-Spektroskopie.
$^{13}$C NMR ($CDCl_3$): δ = 12.7 ($\underline{C}H_3CH_2N$), 25.3, 25.4 (($\underline{C}H_3)_2C$-), 44.5 ($N\underline{C}H_2CH_3$), 55.3 ($OCH_3$), 58.5, 59.3 (quart. C, $(CH_3)_2\underline{C}$-), 68.7, 69.3 ($NCH_2$), 98.0, 98.5, 101.2, 101.5, 103.0, 103.1, 106.2, 106.3, 133.0, 133.1 (tert. Aryl-C), 108.3, 108.4, 112.1, 112.3, 151.6, 151.9, 163.7, 163.9, 166.0, 166.7, 167.4, 168.4, (quart. Aryl-C), 159.2, 160.2, 164.8, 165.5 ($\underline{C}$=N). MS (EI-MS) m/z: 397.3 $(M)^+$, 205, 192 (Isomer I), 233 (Isomer II)

<u>Beispiel 4:</u>

N-1-[4-(Diethylamino)salicyliden]-N'-2-(4-hydroxysalicyliden)-2-methylpropan-1,2-diamin (Struktur I) und N-2-[4-(Diethylamino)salicyliden]-N'-1-(4-methoxysalicyliden)-2-methylpropan-1,2-diamin (Struktur II)

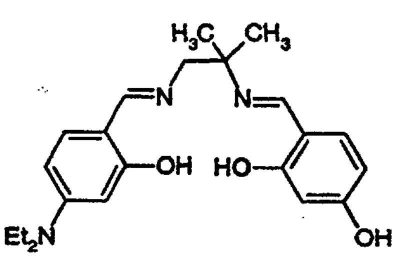

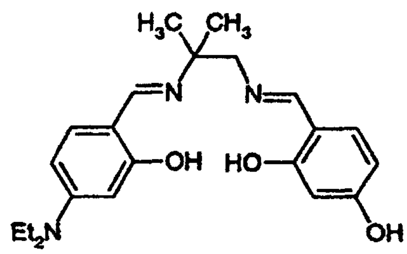

I

II

Eine Lösung von 1.36 g (5.17 mmol) N-Mono[4-(diethylamino)salicyliden]-2-methylpropan-1,2-diamin aus Beispiel 1 in 5 ml Ethanol wird mit 715 mg (5.17 mmol) 4-Hydroxysalicylaldehyd versetzt und die Lösung 3 h auf 60 °C erwärmt. Nach Ende der Reaktion (DC-Kontrolle Essigester/Methanol 9:1) engt man vorsichtig ein und reinigt den Rückstand durch Säulenchromatographie (250 g Kieselgel, Essigester/Methanol 9:1). Ausbeute: 244 mg (12 %), Isomerenmischung.
$^{13}$C NMR ($CD_3OD$): δ = 12.3 ($\underline{C}H_3CH_2N$), 24.1, 24.5 (($\underline{C}H_3)_2C$-), 44.6 ($N\underline{C}H_2CH_3$), 57.3, 58.6 (quart. C), 63.0, 66.3 (=$N\underline{C}H_2$), 98.9, 99.4, 100.1, 104.1, 104.3, 107.7, 134.6, 135.2, 135.8 (tert. Aryl-C), 108.2, 111.3, 111.6, 154.6, 155.1, 164.2, 165.0, 173.9, 175.9 (quart Aryl-C), 158.3, 161.4, 163.5, 166.6 ($\underline{C}$=N).

<u>Beispiel 5:</u>

N-1-[4-(Diethylamino)salicyliden]-N'-2-(salicyliden)-2-methylpropan-1,2-diamin (Struktur I) und N-2-[4-(Diethylamino)salicyliden]-N'-1-(salicyliden)-2-methylpropan-1,2-diamin (Struktur II)

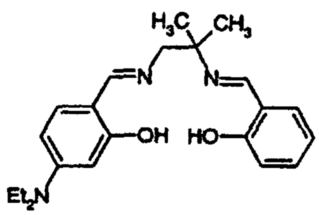

I

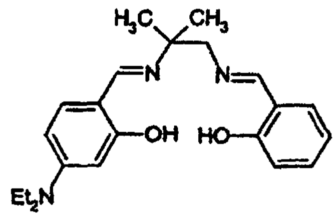

II

Man erwärmt eine Lösung von 13.62 g (0.0517 mol) N-Mono[4-(diethylamino)salicyliden]-2-methylpropan-1,2-diamin aus Beispiel 1 in 50 ml Ethanol auf 50°C und tropft 5.5 ml (6.31 g, 0.0517 mol) Salicylaldehyd innerhalb drei Minuten dazu. Die Temperatur der Lösung steigt dabei um 5 °C. Die Reaktionslösung wird drei Stunden auf Rückfluss gehalten, abkühlen lassen und am Rotationsverdampfer eingeengt. Man erhält 19.31 g Rohgemisch, das die beiden Diastereomeren I und II enthält. Das Rohgemisch wird säulenchromatographisch aufgetrennt (Essigester/Methanol 9:1).
Ausbeute: 4.01g (21 %) I, hellbrauner Feststoff, 1.55 g (8 %) II, hellbraunes Öl. NMR-Daten I
$^{13}C$ NMR ($CD_3OD$): $\delta$ = 12.2 ($\underline{C}H_3CH_2N$), 23.9 (($\underline{C}H_3)_2C$), 44.5 ($N\underline{C}H_2CH_3$), 60.1 (quart. $\underline{C}(CH_3)_2$), 62.0 (=$N\underline{C}H_2$), 99.4, 104.3, 117.0, 118.6, 132.4, 132.8, 135.6 (tert. Aryl-C), 108.3 119.1, 155.2, 162.2 (quart Aryl-C), 162.8, 163.5 ($\underline{C}$=N).
II
$^{13}C$ NMR ($CD_3OD$): $\delta$ = 12.2 ($\underline{C}H_3CH_2N$), 24.7 (($\underline{C}H_3)_2C$-), 44.5 ($N\underline{C}H_2CH_3$), 57.1 (quart. $\underline{C}(CH_3)_2$), 69.3 (=$NCH_2$), 99.6, 104.1, 116.8, 118.9, 132.2, 132.8, 135.9 (tert. Aryl-C), 108.1, 119.1, 155.4, 161.4, 177.0 (quart Aryl-C), 158,0, 168.4 ($\underline{C}$=N).

Beispiel 6:

N-[4-(Dimethylamino)salicyliden]-N'-(salicyliden)-1,2-ethylendiamin

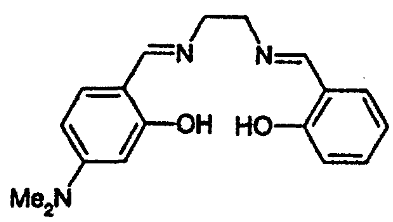

In 30 ml Ethanol werden 5.83 g (35.3 mmol) 4-N-(Dimethylamino)salicylaldehyd und 4.36 g (35.3 mmol) Salicylaldehyd eingetragen und auf 50 °C erwärmt. Man gibt innerhalb zwei Minuten 2.27 ml (2.03 g, 33.6 mmol) Ethylendiamin dazu. Während der Zugabe steigt die Reaktionstemperatur um etwa 15 °C. Die Suspension wird 4 h auf 65 °C erwärmt. Nach dem Abkühlen erstarrt die Reaktionsmasse, man erhält 14.2 g eines dunkelbraunen Feststoffes. Dieser wird mit 100 ml eines Gemisches Essigester/Methanol 9:1 versetzt und 2 h gerührt. Der Feststoff wird abfiltriert und noch zweimal suspendiert. Der übriggebliebene Feststoff wird abfiltiert und das Filtrat wird eingeengt. Der Rückstand wird säulenchromatographisch gereinigt (450 g Kieselgel, Essigester/Methanol 9:1). Ausbeute: 1.3 g (12 %).
$^{13}C$ NMR ($CD_3OD$): $\delta$ = 39.0 ($N\underline{C}H_3$), 53.3, 60.7 (=$N\underline{C}H_2$), 104.3, 117.0, 131.9, 132.1, 132.8, 132.9, 135.0, 135.2, 163.7, 167.3 (tert. Aryl-C), 118.5, 157.3, 161.6, 162.3 (quart. Aryl-C), 163.0, 168.0 (C=N).

Beispiel 7:

N-1-[4-(Dimethylamino)salicyliden]-N'-2-(salicyliden)-2-methylpropan-1,2-diamin (Struktur I) und N-2-[4-(Dimethylamino)salicyliden]-N'-1-(salicyliden)-2-methylpropan-1,2-diamin (Struktur II)

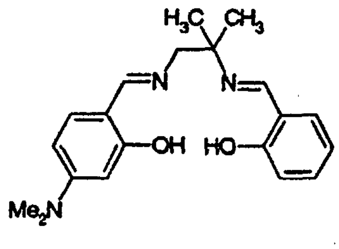

I

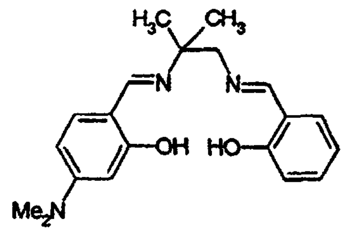

II

Eine Lösung von 7.67 g (30.3 mmol) N-Mono[4-(dimethylamino)salicyliden]-2-methylpropan-1,2-diamin aus Beispiel 2 in 50 ml Ethanol wird auf 50 °C erwärmt. Bei dieser Temperatur tropft man 3.70 g Salicylaldehyd (30.3 mmol) dazu. Die Reaktionslösung wird 1 h auf Rückfluss erhitzt. Die dunkelbraune Suspension wird eingeengt. Man erhält 10.3 g Rohware, die säulenchromatographisch (1 kg Kieselgel, Laufmittel Essigester/Methanol 9:1) gereinigt wird. Ausbeute: 1.7 g (16 %) I gelblicher Feststoff, 0.81 g (8 %) II, bräunlicher Feststoff.
Ausserdem werden 2.9 g (28 %) Isomerenmischung (Zusammensetzung laut $^1$H NMR 1.75 g I, 1.17 g II) isoliert.
Isomer Struktur I:
$^1$H NMR ($CDCl_3$): $\delta$ = 1.40 (s, 6H, $CH_3$), 3.00 (s, 6H, $NCH_3$), 3.60 (m, 2H, =$NC\underline{H_2}$), 6.12, 6.18, 6.83, 6.90, 6.98, 7.21, 7.25 (m, je 1H, tert. Aryl-H), 8.03, 8.32 (s, je 1H, $C\underline{H}$=N).
$^{13}$C NMR ($CDCl_3$): $\delta$ = 25.0 (($\underline{C}H_3)_2C$-), 40.0 ($NCH_3$), 60.2 (quart $\underline{C}(CH_3)_2$), 68.9 (=$NCH_2$), 98.8, 103.5, 117.1, 131.4-132.1 (tert. Aryl-C), 108.8, 153.7 (quart. Aryl-C), 118.8 (tert. Aryl-C und quart. Aryl-C), 161.3 (C=N und quart. Aryl-C), 165.0 (C=N und quart. Aryl-C).
Isomer Struktur II:
$^{13}$C NMR ($CDCl_3$): $\delta$ = 25.5 ($(\underline{C}H_3)_2C$), 40.1 ($NCH_3$), 58.8 (quart. C), 70.7 ($NCH_2$), 99.2, 103.4, 117.0, 118.6, 131.6, 132.3, 132.9 (tert. Aryl-C), 108.9, 154.0, 161.6 (quart Aryl-C), 159.5, 166.5 ($\underline{C}$=N).

Beispiel 8:

N-1-[4-(Dimethylamino)salicyliden]-N'-2-(4-hydroxysalicyliden)-2-methylpropan-1,2-diamin (Struktur I) und N-2-[4-(Dimethylamino)salicyliden]-N'-1-(4-hydroxysalicyliden)-2-methylpropan-1,2-diamin (Struktur II)

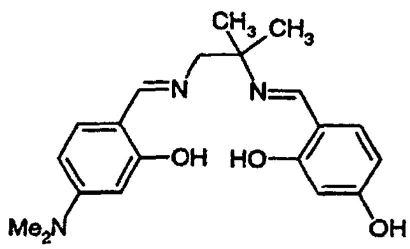

I

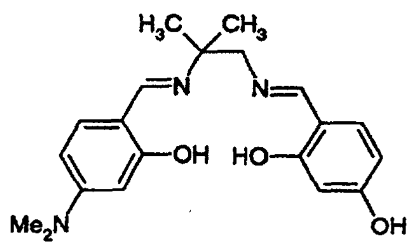

II

Eine Lösung von 7.67 g (30.3 mmol) N-Mono[4-(dimethylamino)salicyliden]-2-methylpropan-1,2-diamin aus Beispiel 2 in 50 ml Ethanol wird auf 55 °C erwärmt. Bei dieser Temperatur werden 4.18 g (30.3 mmol) 2,4-Dihydroxybenzaldehyd (fest) eingetragen. Die Reaktionsmischung wird anschliessend so lange auf Rückfluss erwärmt, bis das Edukt verschwunden ist (DC-Kontrolle, Essigester/Methanol 9:1). Die graubraune Suspension wird eingeengt und am Hochvakuum getrocknet. Man erhält 10.8 g Rohware, die in 50 ml Essigester/Methanol 9:1 suspendiert wird. Die Suspension wird filtriert, eingeengt (1.38 g Rohware) und säulenchromatographisch getrennt (Essigester/Methanol 9:1). Ausbeute: 290 mg (3 %), Isomerenmischung aus I und II.
$^{13}$C NMR (DMSO-$d_6$): $\delta$ = 26.0, 26.1 (($\underline{C}H_3)_2C$-), 40.3 ($NCH_3$), 59.2, 59.7 (quart. C, $\underline{C}(CH_3)_2$), 68.6, 68.9 ($NCH_2$), 99.0, 99.2, 103.4, 103.6, 104.1, 104.3, 107.4, 107.7, 133.6, 134.0, 134.3, 134.6 (tert. Aryl-C), 109.2, 109.4, 112.1, 154.4, 154.5, 162.7, 165.2, 165.5 (quart. Aryl-C), 161.3, 162.0, 168.3, 166.7 ($\underline{C}$=N).

Beispiel 9:

N-1-[4-(Dimethylamino)salicyliden]-N'-2-(4-methoxysalicyliden)-2-methylpropan-1,2-diamin (Struktur I) und N-2-[4-(Dimethylamino)salicyliden]-N'-1-(4-methoxysalicyliden)-2-methylpropan-1,2-diamin (Struktur II)

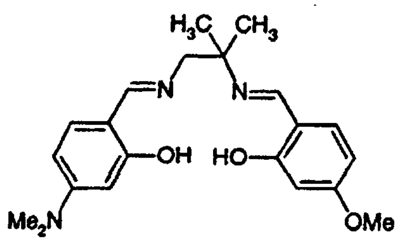

I

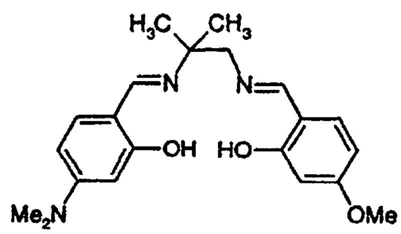

II

Eine Lösung von 7.67 g (30.27 mmol) N-Mono[4-(dimethylamino)salicyliden]-2-methylpropan-1,2-diamin aus Beispiel 2 in 50 ml Ethanol wird auf 50 °C erwärmt. Bei dieser Temperatur trägt man 4.6 g (30.27 mmol) 4-Methoxysalicylaldehyd ein. Man erwärmt 2 h auf Rückfluss, lässt abkühlen und engt die Reaktionslösung ein. Der erhaltene braune Feststoff (12.2 g) wird durch Säulenchromatographie gereinigt (1 kg Kieselgel, Essigester/Methanol 9:1). Ausbeute: 4.69 g (42 %) Isomerenmischung aus I und II.
$^{13}C$ NMR ($CDCl_3$): δ = 25.3 (($\underline{C}H_3)_2C$), 40.3 ($NCH_3$), 55.3 ($OCH_3$), 58.6, 59.2 (quart. C, $\underline{C}(CH_3)_2$), 68.8, 69.2 (=$N\underline{C}H_2$), 98.8, 99.2, 101.2, 101.4, 103.4, 103.5, 106.2, 106.4, 132.8, 133.1 (tert. Aryl-C), 108.7, 108.8, 112.1, 112.3, 153.8, 154.1, 163.7, 164.0, 166.8, 167.5 (quart Aryl-C), 159.5, 160.2, 165.0, 165.5 ($\underline{C}$=N).

Beispiel 10:

N-[4-(Diethylamino)salicyliden]-N'-(4-methoxysalicyliden)-1,2-ethylendiamin

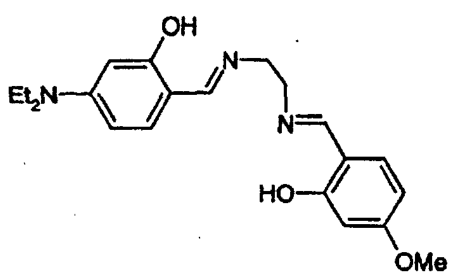

Man legt eine Lösung von 3.87 g (0.0644 mol) Ethylendiamin in 300 ml Ethanol vor und tropft langsam unter Rühren bei Raumtemperatur eine Lösung von 12.45 g (0.0644 Mol) 4-N-(Diethylamino)-salicylaldehyd in 60 ml Ethanol zu. Die Lösung wird 2 h unter Rückfluss erhitzt. Nach Abkühlen auf Raumtemperatur wird eine Lösung aus 9.8 g (0.0644 mol) 4-Methoxysalicylaldehyd in 25 ml Ethanol langsam zugetropft. Anschliessend erwärmt man die Reaktionslösung 1 h auf Rückflusstemperatur. Man lässt langsam abkühlen und rührt 8 h bei Raumtemperatur. Zur Aufarbeitung wird die entstandene gelbe Suspension im Vakuum eingeengt und durch Säulenchromatographie an Kieselgel (Laufmittel:
Essigsäureethylester/Methanol 9:1) gereinigt. Der unsymmetrische Ligand wird als oranges Öl isoliert. Ausbeute: 4.00 g (17 %).
$^{13}C$ NMR ($CDCl_3$): δ = 12.7 ($\underline{C}H_3CH_2N$), 44.4 ($N\underline{C}H_2CH_3$), 55.3 ($OCH_3$), 58.1, 58.7 ($N\underline{C}H_2$), 98.0, 101.2, 103.1, 106.3, 132.9, 133.0 (tert. Aryl-C), 108.3, 112.3, 151.5, 163.5 (quart. Aryl-C), 164.5, 165.4 (C=N).

Beispiel 11:

N-[4-(Diethylamino)salicyliden]-N'-(4-hydroxysalicyliden)-1,2-ethylendiamin

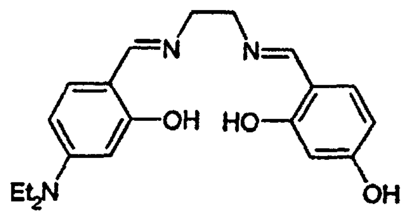

In 30 ml Ethanol werden 6.09 g (31.5 mmol) 4-N-(Diethylamino)salicylaldehyd und 4.35 g (31.5 mmol) 2,4-Dihydroxybenzaldehyd eingetragen und auf 50 °C erwärmt. Man gibt innerhalb zwei Minuten 2 ml (1.80 g, 30 mmol) Ethylendiamin dazu. Während der Zugabe steigt die Reaktionstemperatur um etwa 10 °C. Die Reaktionslösung wird 4 h auf 65 °C erwärmt. Nach dem Abkühlen wird die Lösung einengt, man erhält 14.2 g eines dunkelbraunen Öls. Dieses wird mit 100 ml eines Gemisches Essigester/Methanol 9:1 versetzt und 2 h gerührt. Die Suspension wird filtriert und eingeengt. Der Rückstand wird säulenchromatographisch gereinigt (450 g Kieselgel, Essigester/Methanol 9:1).
Ausbeute: 1.18 g (11 %), oranges Öl.
$^{13}C$ NMR (DMSO-$d_6$): $\delta$ = 13.4 ($\underline{C}H_3CH_2N$), 44.6 ($N\underline{C}H_2CH_3$), 57.9, 58.6 ($=N\underline{C}H_2$), 98.2, 103.5, 103.7, 107.8, 134.0, 134.3 (tert. Aryl-C), 108.6, 112.0, 126.3, 152.0, 162.8, 166.2 (quart. Aryl-C), 165.7, 166.6 (C=N).

<u>Beispiel 12:</u>

N-[4-(Diethylamino)salicyliden]-N'-(salicyliden)-1,2-ethylendiamin

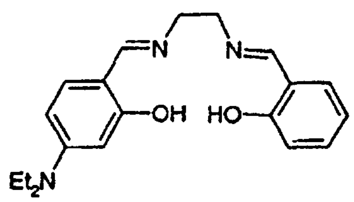

10 g (51.7 mmol) 4-N-(Diethylamino)salicylaldehyd werden mit 6.31 g (51.7 mmol) Salicylaldehyd in 50 ml Ethanol gelöst. Dazu gibt man bei Raumtemperatur 3.1 g (51.7 mmol) Ethylendiamin. Die Lösung erwärmt sich dabei auf ca. 40 °C. Man erhitzt 3 h auf 70 °C und lässt abkühlen. Die Reaktionslösung wird eingeengt, es verbleiben 21.6 g eines rotbraunen Öls. Das Rohprodukt wird säulenchromatographisch (1 kg Kieselgel, Essigester/Methanol 20:1) gereinigt.
Ausbeute: 1.5 g (8.5 %).
$^{13}C$ NMR ($CD_3OD$): $\delta$ = 13.5 ($\underline{C}H_3CH_2N$), 46.0 ($N\underline{C}H_2CH_3$), 53.3, 60.7 ($=N\underline{C}H_2$), 100.5, 105.7, 118.5, 120,3, 132.8, 133.5, 137.0 (tert. Aryl-C), 109.6, 156.6, 164.5, 177.2 (quart. Aromaten), 163.0, 169.5 (C=N).

<u>Beispiel 13:</u>

(R,R)-N-[4-(Diethylamino)salicyliden]-N'-(salicyliden)-1,2-cyclohexandiamin

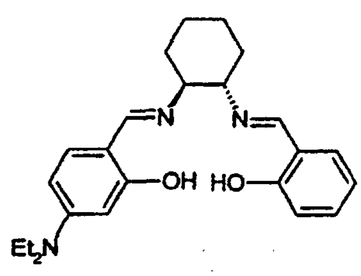

Man löst 0.2 g (0.916 mmol) (R,R)-N-Mono(salicyliden)-1,2-cyclohexandiamin, hergestellt nach *Tetrahedron*

*Letters* **39** (1998) 4199-4202, in 20 ml Ethanol zu einer klaren gelben Lösung. Bei Raumtemperatur wird 177 mg (0.916 mmol) 4-N-(Diethylamino)salicylaldehyd gelöst in 20 ml Ethanol zugetropft. Die dunkelrote Reaktionslösung wird 4 h auf 60 °C erwärmt, danach lässt man auf Raumtemperatur abkühlen und engt vorsichtig am Rotationsverdampfer ein. Man erhält 386 mg eines roten Feststoffes. Dieses Rohprodukt wird durch Säulenchromatographie gereinigt (30 g Kieselgel, Laufmittel: Essigester). Ausbeute: 124.0 mg (34 %) honigfarbene wabenartige Kristalle.
$^{13}C$ NMR ($CDCl_3$): δ = 12.7 ($\underline{C}H_3CH_2N$), 24.2, 24.4, 33.2, (cycl. $CH_2$), 44.4 ($CH_3\underline{C}H_2N$), 70.9, 72.9 (tert. cycl. CH), 97.9, 103.0, 116.7, 118.5, 131.5, 132.0, 132.9 (tert. Aryl-C), 108.2, 118.8 151.3, 164.6, 165.3 (quart. Aryl-C), 161.1, 162.8 ($\underline{C}$=N).

Beispiel 14:

(R,R)-N-[4-(Dimethylamino)salicyliden]-N'-(salicyliden)-1,2-cyclohexandiamin

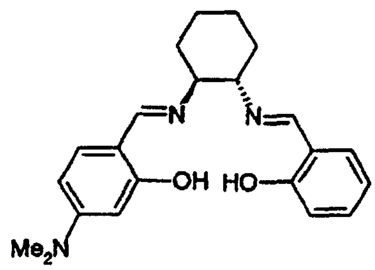

0.5 g (2.29 mmol) (R,R)-N-Mono(salicyliden)-1,2-cyclohexandiamin werden mit 0.378 g (2.29 mmol) 4-N-(Dimethylamino)salicylaldehyd umgesetzt wie im vorigen Beispiel beschrieben. Nach analoger Aufarbeitung erhält man einen gelben Feststoff als Rohprodukt (829 mg), der durch säulenchromatographische Trennung (Kieselgel, Essigester/Methanol 9:1) gereinigt wird. Ausbeute: 318 mg (38 %), hellgelber Feststoff $^{13}C$ NMR ($CDCl_3$): δ = 24.2, 24.4, 33.2. (cycl. $CH_2$), 40.0 (N-$CH_3$), 71.1, 72.9 (tert. cycl. CH), 98.7, 103.4, 116.7, 118.5, 131.5, 132.0, 132.6 (tert. Aryl-C), 108.7, 118.7, 153.6, 161.1 (quart. Aryl-C), 163.2, 164.7 ($\underline{C}$=N).

Beispiel 15:

(R,R)-N-[2-hydroxyacetophenon]-N'-(salicylaldehyd)-1,2-cyclohexandiimin

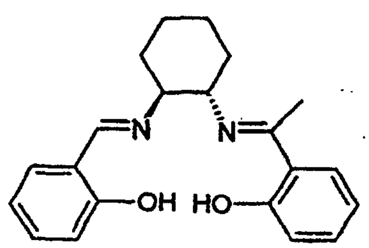

Zu einer Lösung von 0.5 g (2.29 mmol) (R,R)-N-Mono(salicyliden)-1,2-cyclohexandiamin in 50 ml Ethanol tropft man 0.32 g (2.29 mmol) 2-Hydroxyacetophenon, gelöst in 50 ml Ethanol. Man erhitzt 8 Stunden auf Rückflusstemperatur. Nach Abkühlen und Einengen der Reaktionslösung erhält man 714 mg eines braunen Feststoffes. Dieses Rohprodukt wird säulenchromatographisch gereinigt (Laufmittel Toluol/Essigester 3:1). Ausbeute: 215.6 mg (28 %), gelblicher Sirup. $^{13}C$ NMR ($CDCl_3$): δ = 14.7 ($CH_3$), 24.2, 24.3, 32.3, 33.1 (cycl. $CH_2$), 62.3, 73.7 (tert. cycl. CH), 116.8, 117.1, 118.6, 118.7, 128.3, 131.6, 132.3, 132.4 (tert. Aryl-C), 119.2, 160.9, 163.9, 170.8 (quart. Aryl-C), 164.8 ($\underline{C}$=N).

Beispiel 16:

(R,R)-N-Mono[4-(diethylamino)salicyliden]-1,2-cyclohexandiamin

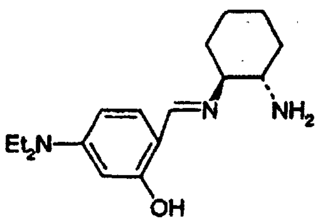

Eine Lösung von 3.95 g (34.55 mmol) trans-1,2-Diaminohexan in 770 ml Chloroform wird mit 50 g Molsieb (4 Å) versetzt und auf -3 °C gekühlt. Bei dieser Temperatur tropft man innerhalb 5 h 6.68 g (34.55 mmol) 4-N-(Diethylamino)salicylaldehyd, gelöst in 250 ml Chloroform, dazu. Nach Zugabe lässt man die Reaktionslösung auf Raumtemperatur erwärmen und 8 h rühren. Der Reaktionsverlauf wird per DC-Kontrolle verfolgt (Laufmittel Essigester/Methanol 9:1). Nach Beendigung der Reaktion wird die Reaktionslösung filtriert und eingeengt. Man erhält 9.9 g (100 %) Rohprodukt, das ohne weitere Reinigung weiter eingesetzt wird.

Beispiel 17:

(R,R)-N-[4-(Diethylamino)salicyliden]-N'-(4-hydroxysalicyliden)-1,2-cyclohexandiamin

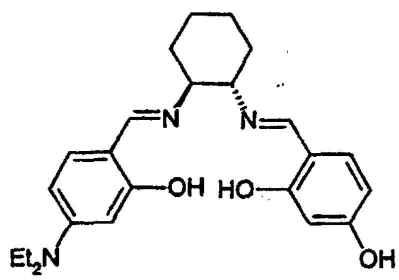

Zu einer Suspension von 2.5 g (8.64 mmol) (R,R)-N-Mono[4-(diethylamino)salicyliden]-1,2-cyclohexandiamin in 200 ml Ethanol tropft man innerhalb 45 Minuten bei Raumtemperatur eine Lösung von 1.19 g (8.64 mmol) 2,4-Dihydroxybenzaldehyd. Die Suspension wird 4 h auf 60 °C erwärmt. Nach dem Abkühlen auf Raumtemperatur wird die erhaltene braunorange Lösung zur Trockne eingeengt. Das Rohprodukt (3.5 g) wird säulenchromatographisch aufgetrennt (Essigester/Methanol 9:1). Ausbeute: 570 mg (16 %), gelboranger Feststoff.
$^{13}C$ NMR ($CDCl_3$): δ = 12.4($\underline{C}H_3CH_2N$), 23.8, 32.7, 32.8 (cycl.-$CH_2$), 43.7 ($CH_3\underline{C}H_2$), 70.2, 70.6 (tert. cycl. CH), 97.1, 102.3, 102.7, 106.7, 132.8, 133.1 (tert. Aryl-C), 107.6, 111.1, 150.7, 161.4, 163.9 (quart. Aryl-C), 163.1, 163.8 ($\underline{C}$=N).

Beispiel 18:

(R,R)-N-[4-(Diethylamino)salicyliden]-N'-(4-methoxysalicyliden)-1,2-cyclohexandiamin

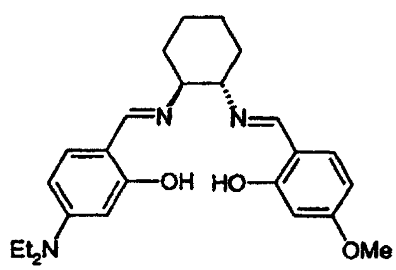

Zu einer Suspension von 2.5 g (8.64 mmol) (R,R)-N-Mono[4-(diethylamino)salicyliden]-1,2-cyclohexandiamin in 200 ml Ethanol tropft man innerhalb 45 Minuten bei Raumtemperatur eine Lösung von 1.3 g (8.64 mmol) 4-Methoxysalicylaldehyd in 200 ml Ethanol. Man erwärmt die Reaktionslösung 4 h auf 60°C. Nach Abkühlen der Reaktionslösung auf Raumtemperatur engt man zur Trockene ein. Die erhaltene Rohware wird säulenchromatographisch gereinigt.

(Essigester/Methanol 9:1). Ausbeute: 500 mg (14 %), rotoranges Öl, das langsam kristallisiert.)
$^{13}C$ NMR ($CDCl_3$): $\delta$ = 12.7 ($\underline{C}H_3CH_2N$), 24.3, 33.2 (cycl.-$CH_2$), 44.4 ($CH_3\underline{C}H_2N$), 55.3 ($OCH_3$), 70.9, 71.5, 71.6 (tert. cycl. CH), 98.0, 101.1, 103.0, 106.1, 106.2, 132.9 (tert. Aryl-C), 108.2, 112.3, 151.3, 165.5 (quart. Aryl-C), 162.9, 163.7 ($\underline{C}$=N).

Synthese der Mangankomplexe:

<u>Beispiel 19:</u>

[N-[4-(Diethylamino)salicyliden]-N'-(4-methoxysalicyliden)-1,2-ethylendiaminato]mangan(III)-chlorid

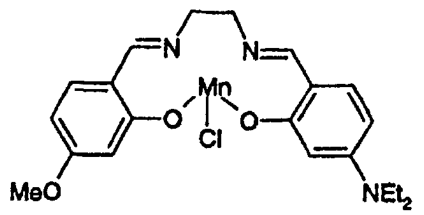

Durchführung: 200 mg (0.541 mmol) Ligand aus Beispiel 10 werden in 11 ml Ethanol gelöst. In diese orange klaren Lösung trägt man 133 mg (0.541 mmol) Mangan-II-acetat-tetrahydrat hinein. Es erfolgt ein Farbumschlag nach dunkelrot und es bildet sich ein Niederschlag. Man erwärmt für 4 h auf 70 °C, dabei geht der Niederschlag in Lösung. Anschliessend dampft man die Reaktionslösung am Rotationsverdampfer bis zur Trockne ein. Man erhält 306 mg Feststoff, der in 11 ml dest. Wasser gelöst wird. Das Produkt wird mit 0.54 g Kochsalz ausgefällt. Man lässt 10 min ausrühren, filtriert und trocknet im Vakuum bei 50 °C.
Ausbeute: 220 mg (89 %), rotschwarzer Feststoff.

<u>Beispiel 20:</u>

[N-2-[4-(Diethylamino)salicyliden]-N'-1-(salicyliden)-2-methylpropan-1,2-diaminato]mangan(III)-chlorid

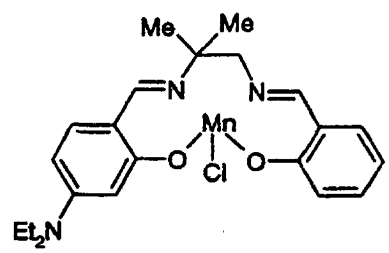

1.2 g (3.25 mmol) Ligand II aus Beispiel 5 werden in 65 ml Ethanol gelöst. Zu dieser gelborangen Lösung gibt man bei Raumtemperatur 0.80 g (3.25 mmol) Mangan-II-acetat-tetrahydrat. Es erfolgt ein Farbumschlag nach rot. Die Reaktionsmischung wird 4 h auf 65-70 °C erwärmt. Nach Einengen der Reaktionsmischung zur Trockne wird mit 65 ml dest. Wasser aufgenommen und der Komplex mit 3.25 g Kochsalz ausgefällt, filtriert und im Hochvakuum bei 50 °C bis zur Massenkonstanz getrocknet. Ausbeute: 1.1 g (74%), rotbrauner Feststoff.

<u>Beispiel 21:</u>

[N-1-[4-(Dimethylamino)salicyliden]-N'-2-(salicyliden)-2-methylpropan-1,2-diamlnato]mangan(III)-chlorid

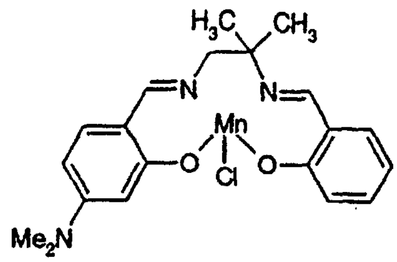

Eine Suspension von 1 g (2.95 mmol) Ligand I aus Beispiel 7 in 60 ml Ethanol wird mit 0.72 g (2.95 mmol) Mangan-II-acetat-tetrahydrat versetzt Die Reaktionsführung und Aufarbeitung (Ausfällung mit 6 g Kochsalz) erfolgt analog der Vorschrift zu Verbindung aus Beispiel 20.
Ausbeute: 924 mg (73 %), rotbrauner Feststoff.

<u>Beispiel 22:</u>

[N-2-[4-(Dimethylamino)salicyliden]-N'-1-(salicyliden)-2-methylpropan-1,2-diaminato]mangan(III)-chlorid

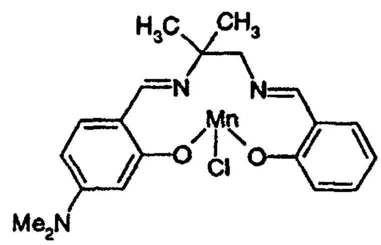

Eine Lösung von 529 mg (1.56 mmol) Ligand II aus Beispiel 7 in 30 ml Ethanol wird mit 380 mg (1.56 mmol) Mangan-II-acetat-tetrahydrat umgesetzt und mit 3.1 g Kochsalz ausgefällt wie im Beispiel 20 beschrieben. Ausbeute: 929 mg. Der Metallkomplex enthält noch Kochsalz und wurde ohne weitere Reinigung eingesetzt.

<u>Beispiel 23:</u>

[N-[4-(Diethylamino)salicyliden]-N'-(4-hydroxysalicyliden)-1,2-ethylendiaminato]-mangan(III)chlorid

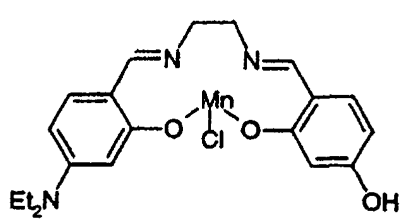

300 mg (0.844 mmol) Ligand aus Beispiel 11 werden in 17 ml Ethanol suspendiert und mit 207 mg (0.844 mmol) Mangan-II-acetat-tetrahydrat versetzt. Es erfolgt ein Farbumschlag nach rot. Die Reaktionsmischung wird innerhalb 30 min auf Rückfluss erhitzt, danach 3 h auf 65 - 70 °C gehalten. Nach dem Einengen der Lösung erhält man 419 mg eines Feststoffes, der in 17 ml dest. Wasser aufgenommen wird. Der Komplex wird durch Eintragen von 0.84 g Kochsalz ausgefällt, filtriert und getrocknet.
Ausbeute: 314 mg (84 %), dunkelroter Feststoff.

<u>Beispiel 24:</u>

[N-1-[4-(Diethylamino)salicyliden]-N'-(4-methoxysalicyliden)-2-methylpropan-1,2-diaminato]mangan(III)-chlorid (Struktur I) und [N-2-[4-(Diethylamino)salicyliden]-N'-1-(4-methoxysalicyliden)-2-methylpropan-1,2-diaminato]mangan(III)chlorid (Struktur II)

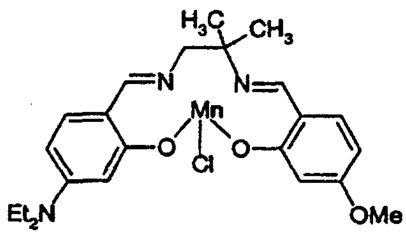

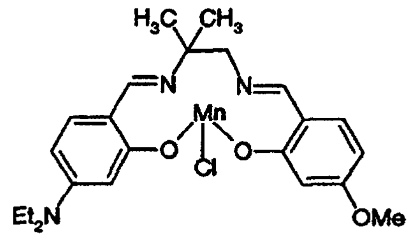

3 g (7.5 mmol) Ligand (isomere Mischung) aus Beispiel 3 werden in 150 ml Ethanol gelöst. Zu dieser hellbraunen, in starker Verdünnung gelben Lösung gibt man 1.85 g (7.5 mmol) Mangan-II-acetat-tetrahydrat. Es erfolgt sofort ein Farbumschlag nach rot. Die klare Lösung erwärmt man für 4 h auf 65-70 °C und engt nach dem Abkühlen ein. Das erhaltene dunkelrote Öl (4.33 g) wird in 150 ml dest. Wasser aufgenommen. Der Komplex wird mit 7.5 g Kochsalz gefällt, filtriert und getrocknet.
Ausbeute: 2.68 g (73 %), dunkelroter Feststoff, Isomerengemisch.

Beispiel 25:

[N-[4-(Diethylamino)salicyliden]-N'-(salicyliden)-1,2-ethylendiaminato]mangan(III)chlorid

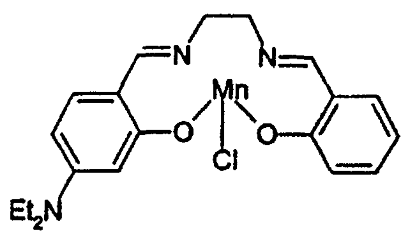

Eine Lösung von 194 mg (0.57 mmol) Ligand aus Beispiel 12 in 10 ml Ethanol wird mit 140 mg (0.57 mmol) Mangan-II-acetat-tetrahydrat versetzt. Es erfolgt ein Farbumschlag von rotbraun nach blutrot. Die Reaktionslösung wird 3 h unter Rückfluss gekocht, über Nacht rühren gelassen und weitere 3 h erwärmt Nach dem Abkühlen engt man zur Trockene ein, nimmt den Rückstand mit 10 ml destilliertem Wasser auf, fällt mit 1.5 g Kochsalz aus, filtriert und trocknet im Hochvakuum. Das Produkt enthält noch Kochsalz und wird ohne weitere Reinigung verwendet.

Beispiel 26:

[N-1-[4-(Diethylamino)salicyliden]-N'-2-(salicyliden)-2-methylpropan-1,2-diaminato]mangan(III)chlorid

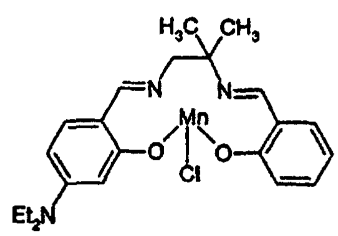

In eine Lösung aus 1.5 g (4.08 mmol) Ligand I aus Beispiel 5 in 80 ml Ethanol trägt man 1.0 g (4.08 mmol) Mangan-II-acetat-tetrahydrat. Die erhaltene Lösung wird 2 Stunden auf 65 °C erwärmt. Nach dem Abkühlen engt man die Lösung zur Trockne ein, nimmt mit 80 ml destilliertem Wasser auf, fällt das Produkt mit 4.1 g Kochsalz aus, filtriert und trocknet im Hochvakuum. Ausbeute: 1.51 g (81 %).

Beispiel 27:

[N-[4-(Dimethylamino)salicyliden]-N'-(salicyliden)-1,2-ethylendiaminato]mangan(III)chlorid

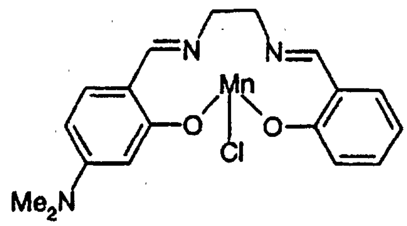

In eine Lösung aus 110 mg (0.353 mmol) Ligand aus Beispiel 6 in 5 ml Ethanol trägt man 86.5 mg (0.353 mmol) Mangan-II-acetat-tetrahydrat. Die erhaltene rotbraune Lösung wird 2 Stunden auf 65 °C erwärmt. Nach dem Abkühlen engt man die Lösung zur Trockne ein, nimmt mit 5 ml destilliertem Wasser auf, fällt das Produkt mit 200 mg Kochsalz aus, filtriert und trocknet im Hochvakuum. Ausbeute: 61 mg (43 %).

Beispiel 28:

(R,R)-N-[4-(dimethylamino)salicyliden]-N'-(4-hydroxysalicyliden)-1,2-cyclohexandiamin

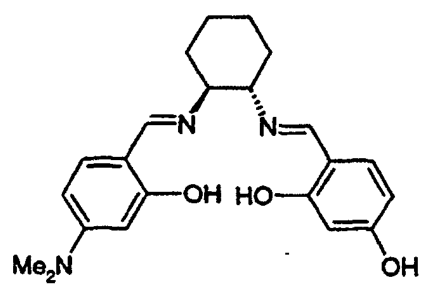

Zu einer Lösung von 2.5 g (9.56 mmol) (R,R)-N-Mono(4-dimethylsalicyliden)-1,2-cyclohexandiamin in 225 ml Ethanol tropft man innerhalb 45 Minuten bei Raumtemperatur eine Lösung von 1.321 g (9.56 mmol) 2,4-Dihydroxybenzaldehyd in 225 ml Ethanol. Die Reaktionslösung wird 4 h auf 60 °C erwärmt. Nach dem Abkühlen auf Raumtemperatur wird die erhaltene rotbraune klare Lösung zur Trockne eingeengt. Das Rohprodukt (ca. 5 g) wird säulenchromatographisch aufgetrennt (Essigester/Methanol 9:1). Ausbeute: 1.09 (30 %, gelboranger Feststoff).
$^{13}$C NMR (DMSO-$d_6$): δ = 23.7, 32.7, 32.8 (cycl.-$CH_2$), 40.0 ($NCH_3$), 70.3, 70.7 (tert. cycl. CH), 97.9, 102.3, 103.2, 106.7, 132.5, 133.1 (tert. Aryl-C), 108.1, 111.1, 153.1, 161.4 (quart. Aryl-C), 163.4, 163.9 (C=N).
$C_{22}H_{27}N_3O_3$ (381.5)

Beispiel 29:

(R,R)-N-[4-(dimethylamino)salicylaldehyd]-N'-(2-hydroxyacetophenon)-1,2-cyclohexandiimin

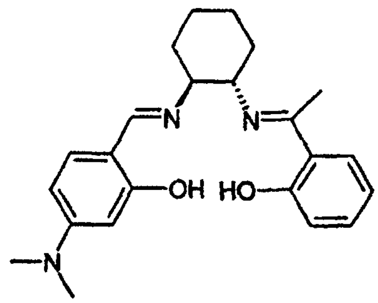

Zu einer Lösung von 2.5 g (9.56 mmol) R,R-Mono[4-N-(dimethylamino)salicyliden-1,2-cyclohexandiamin in 225 ml Ethanol tropft man 1.30 g (9.56 mmol) 2-Hydroxyacetophenon, gelöst in 225 ml Ethanol. Man erhitzt 8 Stunden auf 60°C. Die erhaltene rotbraune klare Lösung wird weitere 4 Stunden bei Raumtemperatur gerührt und im Hochvakuum eingeengt. Man erhält Rohprodukt (3.6 g, dunkelrotes Öl), das säulenchromatographisch gereinigt wird (Laufmittel Essigester/Methanol 9:1). Ausbeute: 1.60 g (44 %), rotoranger Schaum.
$^{13}C$ NMR ($CDCl_3$): δ = 14.7 ($CH_3$), 24.2, 24.3, 32.4, 33.2 (cycl. $CH_2$), 40.0 ($NCH_3$), 62.3, 72.2 (tert. cycl. CH), 98.6, 103.4, 116.8, 118.6, 128.3, 132.3, 132.7 (tert. Aryl-C), 108.6, 119.1, 153.6, 164.3, 170.9 (quart. Aryl-C), 163.2 (<u>C</u>=N).
$C_{23}H_{29}N_3O_2$ (379.5)

<u>Beispiel 30:</u>

N-Mono[4-(diethylamino)salicyliden]-1,2-phenylendiamin

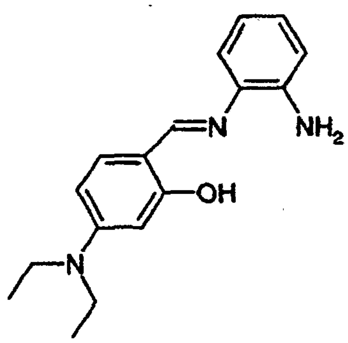

Zu einerLösung von 1.927 g (17.64 mmol) 1,2-Phenylendiamin in 18 ml Ethanol trägt man bei 5 °C portionsweise 3.479 g (17.64 mmol) 4-(N,N-Diethylamino)-salicylaldehyd ein, wobei die Temperatur nicht über 10 °C steigen sollte. Die erhaltene dunkelrote Suspension wird 8 Stunden bei Raumtemperatur gerührt und im Vakuum eingeengt. Man erhält 6.34 g Rohprodukt, das säulenchromatographisch (Laufmittel n-Hexan/Essigester 65:35) getrennt wird. Ausbeute: 1.273 g (26 %), goldgelbe Kristalle. $^{13}C$ NMR ($CDCl_3$): δ = 12.7 ($CH_3$), 44.6 (CH2), 97.6, 103.7, 115.4, 118.2, 118.8, 126.6, 133.7 (tert. Aryl-C), 109.3, 136.4, 140.4, 151.6, 163.2 (quart. Aryl-C), 160.9 (C=N).

<u>Beispiel 31:</u>

N-[4-(diethylamino)salicyliden]-N'-(salicyliden)-1,2-phenylendiamin

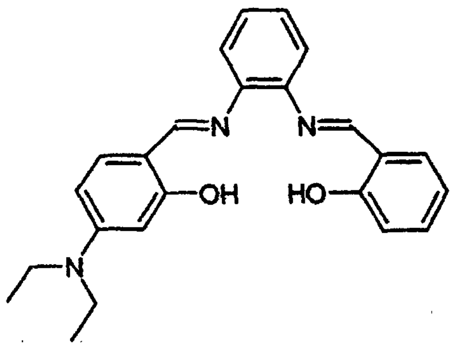

Zu einer gelbbraune Suspension von 0.3 g (1.06 mmol) N-Mono[4-(diethylamino)salicyliden]-1,2-phenylendiamin aus Beispiel 30 in 2 ml Ethanol wird bei 60 °C 129 mg (112 µl, 1.06 mmol) Salicylaldehyd zugetropft. Die Reaktionslösung wird 5 Stunden bei 75 °C gerührt. Die rotbraune Reaktionslösung wird nach dem Abkühlen auf Raumtemperatur eingeengt. Der Rückstand wird säulenchromatographisch (Laufmittel n-Hexan/Essigester 65:35) aufgereinigt. Ausbeute: 139 mg (34 %), farbloses Öl.
$^{13}C$ NMR ($CDCl_3$): δ = 11.7 ($\underline{C}H_3CH_2N$), 44.4 ($CH_3\underline{C}H_2N$), 97.0, 102.4 (tert. Aryl-H), 116.5-119.0, 125.1, 126.6, 130.0-132.0 (tert. Aryl-C), 108.3, 118.3, 141.1, 141.4, 151.2, 160.3 (quart. Aryl-C), 159.5, 162.4 ($\underline{C}$=N).
$C_{24}H_{25}N_3O_2$ (387.5)

<u>Beispiel 32:</u>

Darstellung eines Gemisches aus unsymmetrischen und symmetrischen Mangan-III-Salenkomplexen

Die beschriebenen unsymmetrischen Salenkomplexe können auch ohne aufwendige Reinigung als Mischung verschiedener Metallkomplexe eingesetzt werden.
4.74 g Rohgemisch aus Beispiel 5 werden mit 250 ml Ethanol verdünnt. Man erhält eine klare braune Lösung. Es werden 3.17 g (12.9 mmol) Mangan-II-acetat-tetrahydrat zugegeben, dabei erfolgt ein Farbumschlag nach rot. Man lässt 4 Stunden bei 65- 70 °C abreagieren und engt zur Trockne ein. Der erhaltene Feststoff wird in 250 ml destilliertem Wasser aufgenommen und mit 26 g Kochsalz ausgesalzen und im Vakuum getrocknet. Die Rohmischung wird direkt appliziert. Das Rohprodukt enthält die unsymmetrischen Metallkomplexe aus den Beispielen 20 und 26 und daneben noch die Metallkomplexe des symmetrischen Liganden der Struktur:

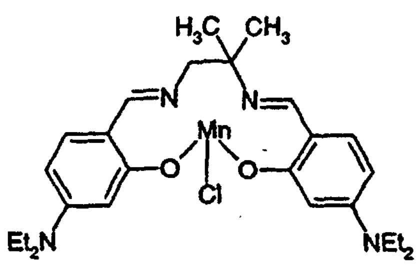

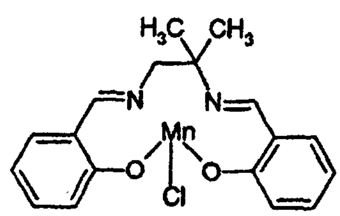

<u>Beispiel 33</u>: Zur Untersuchung der Wirksamkeit der Katalysatoren wird die DTI- Wirksamkeit bestimmt.

Die DTI (Dye transfer inhibition/ Farbstofftransferverhinderung) -Wirksamkeit a ist als folgender Prozentsatz definiert.

$$a = ([Y(E) - Y(A)] / [Y(W) - Y(A)]) * 100$$

wobei Y(W), Y(A) und Y(E) die CIE-Helligkeiten des weissen Materials, des ohne Katalysatorzusatz behandelten Materials und des mit Katalysatorzusatz behandelten Materials in dieser Reihenfolge bedeuten. *a=0* charakterisiert ein vollständig nutzloses Produkt, dessen Zusatz zur Waschlauge dem Farbstofftransfer freien Lauf lässt. *a=100%* dagegen entspricht einem perfekten Katalysator, der die Anfärbung des Weissmaterials vollständig unterbindet.

Zur Ermittlung der Prüfdaten wird das folgende Testsystem verwendet: 7,5 g weisses Baumwollgewebe werden in 80ml Waschlauge behandelt. Diese Lauge enthält das Standardwaschmittel ECE phosphatfrei (456 IEC) EMPA, Schweiz, in einer Konzentration von 7,5g/l, 8,6 mmol/l $H_2O_2$ und eine Lösung des Testfarbstoffes. Der Waschprozess findet in einem Becher in einem LINITEST-Apparat während 30 Min. bei 40°C statt. Die Katalysatoren werden dabei standardmässig in den angegebenen Konzentrationen eingesetzt.

Folgende kommerziell erhältlichen Farbstoffe werden als Testfarbstoffe eingesetzt:

| | | |
|---|---|---|
| Farbstoff 1 | (F1) | Direct Brown 172 |
| Farbstoff 4 | (F4) | Reactive Blue 238 |
| Farbstoff 6 | (F6) | Reactive Black 5 |
| Farbstoff 8 | (F8) | Direct Blue 71 |
| Farbstoff 9 | (F9) | Direct Black 22 |
| Farbstoff 10 | (F10) | Anionic Blue 113 |
| Farbstoff 13 | F(13) | Disperse Violet 1 |
| Farbstoff 14 | (F14) | Reactive Blue 19 |

Die Reflexionsspektren der Muster wurden mit einem SPECTRAFLASH 2000 gemessen und gemäss Standardprozedur nach CIE in Helligkeiten (D65/10) transformiert.

Die folgende Tabelle zeigt die Ergebnisse mit dem gemäss Beispiel 24 erhaltenen Katalysator (Mn-Komplex). Es sind die DTI-Effekte (a) in Funktion der Katalysatorkonzentration, bei Einsatzbedingungen wie vorstehend beschrieben dargestellt.

Tabelle 1

| | **DTI-Effekt (a)** | |
|---|---|---|
| **Katalysatorkonzentration µmol/l** | **Farbstoff 1** | **Farbstoff 2** |
| 5 | 62 | 76 |
| 20 | 85 | 90 |
| 30 | 89 | 90 |
| 50 | 91 | 91 |

Die folgende Tabelle zeigt, dass der gemäss Beispiel 24 erhaltene Katalysator sehr wirksam das Wiederaufziehen von Farbstoffen verschiedener Klassen verhindert. Die hier angegebenen Werte beziehen sich auf eine Katalysatorkonzentration von 50µmol/l und experimentelle Bedingungen wie vorstehend beschrieben.

Tabelle 2

| **Testfarbstoff** | **Farbstoffkonzentration mg/l** | **DTI-Effekt (a)** |
|---|---|---|
| Direct Brown 172 250% | 10 | 91 |
| Reactive Blue 238 100% | 6 | 91 |
| Reactive Black 5 133% | 12 | 95 |
| Direct Black 022 400% | 6 | 85 |
| Reaktiv Black 19 (Spezial) 100% | 20 | 100 |
| Anionic Blue 113 180% | 6 | 99 |
| Disperse Violet 1 100% | 6 | 86 |

Der Katalysator zeichnet sich im weiteren dadurch aus, dass selbst bei einer Einsatztemperatur von lediglich 20°C der Löwenanteil der bei 40°C beobachteten Schutzwirkung bestehen bleibt.

Tabelle 3:

| | DTI-Effekt (a) | |
|---|---|---|
| **Katalysatorkonzentration μmol/l** | **Farbstoff 1** | **Farbstoff 2** |
| 5 | 57 | 78 |
| 20 | 80 | 89 |
| 30 | 84 | 90 |
| 50 | 85 | 87 |

Der Katalysator weist eine akzeptable Schadenbilanz gegenüber gefärbtem Waschgut auf. Hinsichtlich Farbstoffschädigung wird selbst bei Verwendung von als sehr empfindlich bekannten Farbstoffen nur ein Abbau von derselben Grössenordnung wie mit dem TAED aktivierten Bleichsystem beobachtet. Letzteres gilt im Bereich der Sauerstoffbleiche als Stand der Technik mit einer akzeptierten Schaden/Nutzen-Bilanz. Bei Einsatz wie vorstehend beschrieben, wird nach fünffacher Behandlung folgender prozentualer Farbstoffverlust festgestellt.

Tabelle 4

| | Farbstoffverlust % | |
|---|---|---|
| **Tesfärbung** | **Katalysator 50μmol/l** | **TAED** |
| Vat Brown 1 | 11 | 2 |
| Reactive Brown 17 | 16 | 15 |
| Reactive Red 123 | 14 | 13 |
| Direct Blue 85 | 22 | 14 |

Der Katalysator weist hinsichtlich Faserschädigung an gefärbten Materialien eine bessere Bilanz auf als das zitierte TAED-System. Bei Einsatz wie vorstehend beschrieben, wird nach fünffacher Behandlung folgende relative DP-Emiedrigung festgestellt.

Tabelle 5:

| | relative DP-Erniedrigung % | |
|---|---|---|
| **Testfärbung** | **Katalysator 50μmol/l** | **TAED** |
| Reactive Brown 017 | 2 | 5 |
| Vat Brown 001 | 9 | 19 |
| Reactive Red 123 | 4 | 7 |
| Direct Blue 085 | 10 | 15 |

Beispiel 34:

Führt man das DTI-Screening mit den folgenden isolierten Liganden durch, die vor dem Screening nach einem in-situ Verfahren in die Mn(III)-Komplexe übergeführt wurden, so erhält man die in Tabelle 6 verzeichneten Ergebnisse.

Tabelle 6:

| Mangankomplexe der Liganden aus Beispiel Nr. | a (%) 10 μM (F1) | a (%) 20 μM (F1) | a (%) 10 μM (F4) | a (%) 20 μM (F4) |
|---|---|---|---|---|
| **4** | 70 | 77 | 74 | 84 |
| **8** | 74 | 81 | 77 | 79 |

Tabelle 6: (fortgesetzt)

| Mangankomplexe der Liganden aus Beispiel Nr. | a (%) 10 μM (F1) | a (%) 20 μM (F1) | a (%) 10 μM (F4) | a (%) 20 μM (F4) |
|---|---|---|---|---|
| **9** | 72 | 79 | 75 | 80 |
| **13** | | 76 | | 53 |
| **14** | 82 | 85 | 84 | 88 |
| **15** | | 73 | | |
| **17** | 86 | 86 | 87 | 86 |
| **18** | 85 | 88 | 80 | 90 |
| **28** | | 86 | | 90 |
| **30** | | 80 | | 80 |
| **31** | | 90 | | 85 |

Tabelle 7 zeigt die Ergebnisse, wenn man das DTI-Screening mit den isolierten Mn-Komplexen durchführt.

Tabelle 7:

| Mangankomplexe aus Beispiel Nr. | a (%) 10μM (F1) | a (%) 20μM (F1) | a (%) 10μM (F4) | a (%) 20 μM (F4) |
|---|---|---|---|---|
| **19** | 78 | 87 | 85 | 93 |
| **20** | 86 | 91 | 94 | 87 |
| **21** | | 80 | | 62 |
| **22** | | 90 | | 96 |
| **23** | 62 | 89 | 82 | 95 |
| **24** | 81 | 88 | 95 | 94 |
| **25** | 80 | 89 | 90 | 95 |
| **26** | 77 | 81 | 83 | 86 |
| **27** | 78 | 77 | 54 | 72 |

Beispiel 35:

Die beschriebenen unsymmetrischen Salenkomplexe zeigen auch bei verringerter Peroxidkonzentration eine ausgezeichnete Wirkung. Senkt man die Konzentration von 8.6 mM auf 0.17 mM Peroxid ab, so bleibt die DTI-Wirkung vollständig erhalten. Die folgende Tabelle 8 zeigt die Wirkung des Metallkomplexes aus Beispiel 20 mit reduzierter Peroxidmenge.

Tabelle 8:

| Farbstoff | a [%] 0.17 mM $H_2O_2$ | a [%] 0 mM $H_2O_2$ | a [%] 8.6 mM $H_2O_2$ |
|---|---|---|---|
| | | | |
| F1 | 82 | 48 | 90 |
| F4 | 93 | 55 | 95 |
| F6 | 93 | 54 | 95 |
| F8 | 79 | 1 | 75 |
| F9 | 83 | 48 | 85 |
| F10 | 90 | 71 | 95 |

Tabelle 8: (fortgesetzt)

| Farbstoff | a [%] 0.17 mM $H_2O_2$ | a [%] 0 mM $H_2O_2$ | a [%] 8.6 mM $H_2O_2$ |
|---|---|---|---|
| F13 | 89 | 65 | 85 |
| F14 | 93 | 31 | 95 |

## Patentansprüche

**1.** Verfahren zur Verhinderung des Wiederaufziehens von migrierenden Farbstoffen in einer Waschflotte, **dadurch gekennzeichnet, dass** man der Waschflotte, die ein peroxidhaltiges Waschmittel enthält, 0,5 bis 150 mg pro Liter Waschflotte einer oder mehrerer Verbindungen der Formel

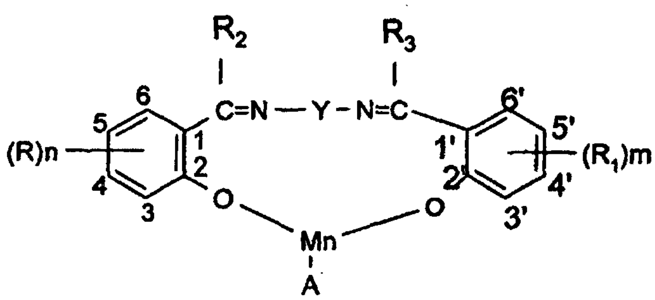

(1)

zusetzt, worin

- n 0, 1, 2 oder 3,
- m 1, 2 oder 3,
- A ein Anion;
- Y ein linearer oder verzweigter Alkylenrest der Formel $-[C(R_5)_2]_r-$, wobei r eine ganze Zahl von 1 bis 8 und die $R_5$-Reste unabhängig voneinander Wasserstoff oder $C_1$-$C_4$-alkyl bedeuten;
  -CX=CX- , worin X Cyano, lineares oder verzweigtes $C_1$-$C_8$-alkyl oder Di(lineares oder verzweigtes $C_1$-$C_8$-alkyl)-amino,
  $-(CH_2)_q-NR_4-(CH_2)_q-$, worin $R_4$ Wasserstoff oder lineares oder verzweigtes $C_1$-$C_4$-alkyl und q 1, 2, 3 oder 4; oder
  ein 1,2-Cyclohexylenrest der Formel:

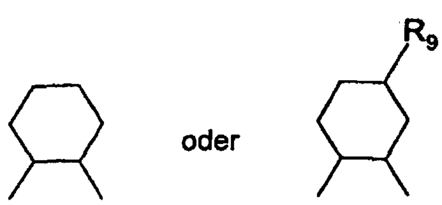

oder ein 1,2-Arylrest der Formel

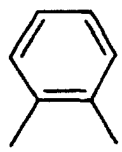

oder

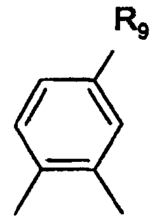

worin $R_9$ $SO_3H$, $CH_2OH$ oder $CH_2NH_2$ ist,
R und $R_1$ unabhängig voneinander Cyano, Halogen, $OR_5$ oder $COOR_5$ worin $R_5$ Wasserstoff oder lineares oder verzweigtes $C_1$-$C_4$-alkyl ist, Nitro, lineares oder verzweigtes $C_1$-$C_8$-alkyl, lineares oder verzweigtes teilfluoriertes oder perfluoriertes $C_1$-$C_8$-alkyl, $NHR_6$ oder $NR_6R_7$ worin $R_6$ und $R_7$ gleich oder verschieden sind und je lineares oder verzweigtes $C_1$-$C_{12}$-alkyl bedeuten oder worin $R_6$ und $R_7$ zusammen mit dem sie verbindenden N-Atom einen 5-, 6-oder 7-gliedrigen Ring bilden, der weitere Heteroatome enthalten kann, oder lineares oder verzweigtes $C_1$-$C_8$-alkyl-$R_8$, worin $R_8$ ein Rest $OR_5$,$COOR_5$ oder $NR_6R_7$ mit den vorstehend genannten Bedeutungen oder $NH_2$ bedeutet, oder -$N^{\oplus}R_4R_6R_7$, worin $R_4$, $R_6$ und $R_7$ die vorstehend genannten Bedeutungen haben,
$R_2$ und $R_3$ unabhängig voneinander Wasserstoff, lineares oder verzweigtes $C_1$-$C_4$ alkyl oder unsubstituiertes Aryl oder Aryl, das durch Cyano, Halogen, $OR_5$ oder $COOR_5$ worin $R_5$ Wasserstoff oder lineares oder verzweigtes $C_1$-$C_4$-alkyl ist, Nitro, lineares oder verzweigtes $C_1$-$C_8$-alkyl, $NHR_6$ oder $NR_6R_7$ worin $R_6$ und $R_7$ gleich oder verschieden sind und die vorstehend angegebenen Bedeutungen haben, oder lineares oder verzweigtes $C_1$-$C_8$-alkyl-$R_8$, worin $R_8$ ein Rest $OR_5$,$COOR_5$ oder $NR_6R_7$ mit den vorstehend genannten Bedeungen
oder $NH_2$ bedeutet, oder -$N^{\oplus}R_4R_6R_7$, worin $R_4$, $R_6$ und $R_7$ die vorstehend genannten Bedeutungen haben, substituiert ist,
mit der Bedingung, dass R und $R_1$ nicht die gleiche Bedeutung haben, falls n und m identisch sind.

2. Verfahren gemäss Anspruch 1, **dadurch gekennzeichnet, dass** man 1,5 bis 75 mg pro Liter Waschflotte einer oder mehrerer Verbindungen der Formel (1) zusetzt.

3. Verfahren gemäss Anspruch 2, **dadurch gekennzeichnet, dass** man 7,5 to 40 mg pro Liter Waschflotte einer oder mehrerer Verbindungen der Formel (1) zusetzt.

4. Verfahren gemäss einem der Ansprüche 1 bis 3, **dadurch gekennzeichnet, dass** das Anion A Halogenid, Perchlorat, Sulfat, Nitrat, Hydroxid, $BF_4^-$, $PF_6^-$, Carboxylat, Triflat oder Tosylat ist.

5. Verfahren gemäss Anspruch 4, **dadurch gekennzeichnet, dass** das Anion A Chlorid oder Acetat ist.

6. Verfahren gemäss einem der Ansprüche 1 bis 5, **dadurch gekennzeichnet, dass** Y einen Rest der Formel $-(CH_2)_r-$, wobei r eine ganze Zahl von 1 bis 8 bedeutet oder der Formel $-C(R_5)_2-(CH_2)_p-C(R_5)_2-$ worin p eine Zahl von 0 bis 6 und $R_5$ Wasserstoff oder $C_1$-$C_4$-alkyl bedeutet.

7. Verfahren gemäss Anspruch 6, **dadurch gekennzeichnet, dass** Y ein Rest der Formel - $(CH_2)_r$- ist, wobei r eine ganze Zahl von 1 bis 4 bedeutet, oder der Formel $-(CR_5)_2-(CR_5)_2-$, worin $R_5$ unabhängig voneinander Wasserstoff oder Methyll bedeutet.

8. Verfahren gemäss einem der Ansprüche 1 bis 7, **dadurch gekennzeichnet, dass** Halogen Chlor, Brom oder Fluor bedeutet.

9. Verfahren gemäss einem der Ansprüche 1 bis 8, **dadurch gekennzeichnet, dass** Halogen Chlor bedeutet.

10. Verfahren gemäss einem der Ansprüche 1 bis 9, **dadurch gekennzeichnet, dass** n und/oder m 1 ist, R und/oder $R_1$ eine der angegebenen Bedeutungen mit Ausnahme von Nitro und $COOR_5$ aufweisen und sich in 4-Stellung des jeweiligen Benzolrings befinden.

11. Verfahren gemäss einem der Ansprüche 1 bis 10, **dadurch gekennzeichnet, dass** n und/oder m 1 ist, R und/ oder $R_1$ Nitro oder $COOR_5$ bedeuten und sich in 4-Stellung des jeweiligen Benzolrings befinden, wobei $R_1$ Wasserstoff oder lineares oder verzweigtes $C_1$-$C_4$-alkyl ist.

**12.** Verfahren gemäss einem der Ansprüche 1 bis 11, **dadurch gekennzeichnet, dass** n und oder m 2 ist, R und/oder $R_1$ eine der angegebenen Bedeutungen mit Ausnahme von Nitro und $COOR_5$ aufweisen und sich in 4,6-Stellung des jeweiligen Benzolrings befinden.

**13.** Verfahren gemäss einem der Ansprüche 1 bis 12, **dadurch gekennzeichnet, dass** n und oder m 2 ist, R und/ oder $R_1$ Nitro oder $COOR_5$ bedeuten und sich in 3,5-Stellung des jeweiligen Benzolrings befinden.

**14.** Verfahren gemäss einem der Ansprüche 1 bis 13, **dadurch gekennzeichnet, dass** R und $R_1$ Nitro, $OR_5$, $COOR_5$ oder $N(R_5)_2$ bedeuten, wobei $R_5$ Wasserstoff oder $C_1$-$C_4$-alkyl ist.

**15.** Verfahren gemäss einem der Ansprüche 1 bis 14, **dadurch gekennzeichnet, dass** R und $R_1$ Nitro, $OR_5$, $COOR_5$ oder $N(R_5)_2$ bedeuten, wobei $R_5$ Wasserstoff, Methyl oder Ethyl ist.

**16.** Verbindungen der Formel

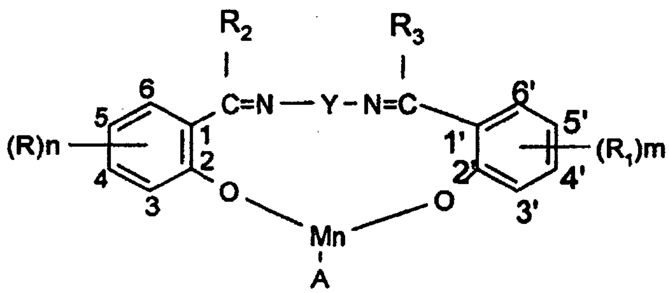

(1a)

dar, worin

n 0, 1, 2 oder 3,
m 1, 2 oder 3,
A ein Anion;
Y ein linearer oder verzweigter Alkylenrest der Formel $-[C(R_5)_2]_r-$, wobei r eine ganze Zahl von 1 bis 8 und die $R_5$-Reste unabhängig voneinander Wasserstoff oder $C_1$-$C_4$-alkyl bedeuten;
-CX=CX- worin X Cyano, lineares oder verzweigtes $C_1$-$C_8$-alkyl oder Di(lineares oder verzweigtes $C_1$-$C_8$-alkyl)-amino,
$-(CH_2)_q-NR_4-(CH_2)_q-$, worin $R_4$ Wasserstoff oder lineares oder verzweigtes $C_1$-$C_4$ alkyl und q 1, 2, 3 oder 4; oder
ein 1,2-Cyclohexylenrest der Formel:

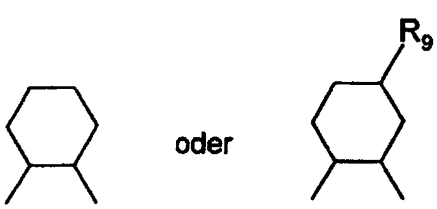

oder ein 1,2-Arylrest der Formel

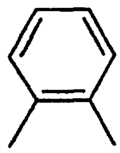 oder 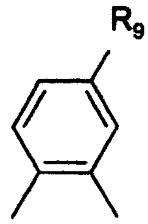

worin $R_9$ $SO_3H$, $CH_2OH$ oder $CH_2NH_2$ ist,
R und $R_1$ unabhängig voneinander Cyano, Halogen, $OR_5$ oder $COOR_5$ worin $R_5$ Wasserstoff oder lineares oder verzweigtes $C_1$-$C_4$-alkyl ist, Nitro, lineares oder verzweigtes $C_1$-$C_8$-alkyl, lineares oder verzweigtes teilfluoriertes oder perfluoriertes $C_1$-$C_8$-alkyl, $NHR_6$ oder $NR_6R_7$ worin $R_6$ und $R_7$ gleich oder verschieden sind und je lineares oder verzweigtes $C_1$-$C_{12}$-alkyl bedeuten oder worin $R_6$ und $R_7$ zusammen mit dem sie verbindenden N-Atom einen 5-, 6-oder 7-gliedrigen Ring bilden, der weitere Heteroatome enthalten kann, oder lineares oder verzweigtes $C_1$-$C_8$-alkyl-$R_8$, worin $R_8$ ein Rest $OR_5$, $COOR_5$ oder $NR_6R_7$ mit den vorstehend genannten Bedeungen oder $NH_2$ bedeutet, oder -$N^{\oplus}R_4R_6R_7$, worin $R_4$, $R_6$ und $R_7$ die vorstehend genannten Bedeutungen haben,
$R_2$ und $R_3$ unabhängig voneinander Wasserstoff, lineares oder verzweigtes $C_1$-$C_4$ alkyl oder unsubstituiertes Aryl oder Aryl, das durch Cyano, Halogen, $OR_5$ oder $COOR_5$ worin $R_5$ Wasserstoff oder lineares oder verzweigtes $C_1$-$C_4$-alkyl ist, Nitro, lineares oder verzweigtes $C_1$-$C_8$-alkyl, $NHR_6$ oder $NR_6R_7$ worin $R_6$ und $R_7$ gleich oder verschieden sind und je lineares oder verzweigtes $C_1$-$C_{12}$-alkyl bedeuten oder worin $R_6$ und $R_7$ zusammen mit dem sie verbindenden N-Atom einen 5-, 6- oder 7-gliedrigen Ring bilden, der weitere Heteroatome enthalten kann, oder lineares oder verzweigtes $C_1$-$C_8$-alkyl-$R_8$, worin $R_8$ ein Rest $OR_5$,$COOR_5$ oder $NR_6R_7$ mit den vorstehend genannten Bedeungen oder $NH_2$ bedeutet, oder -$N^{\oplus}R_4R_6R_7$, worin $R_4$, $R_6$ und $R_7$ die vorstehend genannten Bedeutungen haben, substituiert ist,
mit der Bedingung, dass R und $R_1$ nicht die gleiche Bedeutung haben, falls n und m identisch sind und $R_2$ und $R_3$ beide Wasserstoff bedeuten und dass von den Resten $R_2$ und $R_3$ nicht der eine Wasserstoff und der andere Phenyl bedeutet.

**17.** Verbindungen gemäss Anspruch 16, **dadurch gekennzeichnet, dass**

| | |
|---|---|
| n und m | jeweils unabhängig voneinander 1, 2 oder 3, |
| A | Chlorid, Acetat, Triflat oder Tosylat, |
| Y | einen Rest der Formel -$(CH_2)_r$-, wobei r eine ganze Zahl von 1 bis 8 bedeutet oder der Formel -C$(R_5)_2$-$(CH_2)_p$-C$(R_5)_2$- worin p eine Zahl von 0 bis 6 und $R_5$ Wasserstoff oder $C_1$-$C_4$-alkyl bedeutet. |
| R und $R_1$ | Nitro, $OR_5$, $COOR_5$ oder $N(R_5)_2$ bedeuten, wobei $R_5$ Wasserstoff oder $C_1$-$C_4$-alkyl ist. |

**18.** Verbindungen gemäss Anspruch 17, **dadurch gekennzeichnet, dass**

| | |
|---|---|
| n und m | jeweils unabhängig voneinander 1, 2 oder 3, |
| A | Chlorid, Acetat, Triflat oder Tosylat, |
| Y | einen Rest der Formel -$(CH_2)_r$-, wobei r eine ganze Zahl von 1 bis 8 bedeutet oder der Formel -C$(R_5)_2$-$(CH_2)_p$-C$(R_5)_2$- worin p eine Zahl von 0 bis 6 und $R_5$ Wasserstoff oder $C_1$-$C_4$-alkyl bedeutet. |
| R und $R_1$ | Nitro, $OR_5$, $COOR_5$ oder $N(R_5)_2$ bedeuten, wobei $R_5$ Wasserstoff, Methyl oder Ethyl ist. |

**19.** Verbindungen der Formel

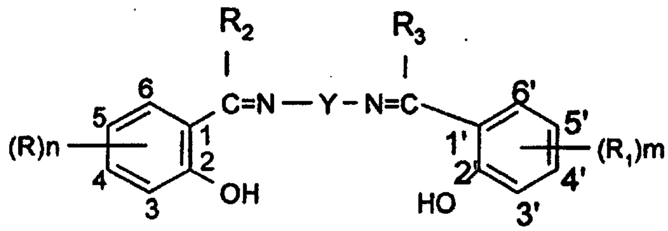

(2)

worin R, $R_1$, $R_2$, $R_3$, Y, n und m die unter der Formel (1a) angegebene Bedeutung haben.

**20.** Verfahren zur Herstellung von Verbindungen der Formel (2) gemäss Anspruch 19, **dadurch gekennzeichnet, dass** man ein Diamin der Formel $H_2N\text{-}Y\text{-}NH_2$ zunächst mit einem Aldehyd oder Keton der Formel

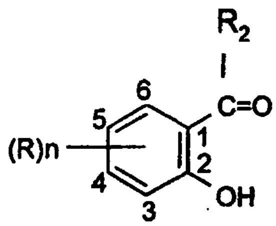

(3)

und danach mit einem Aldehyd oder Keton der Formel

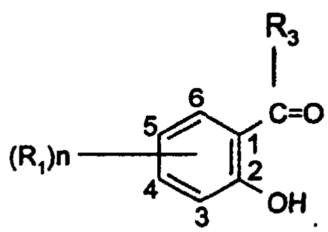

(4)

umsetzt, wobei R, $R_1$, $R_2$, $R_3$, n und m die unter der Formel (1) angegebenen Bedeutungen aufweisen.

**21.** Verbindungen der Formel

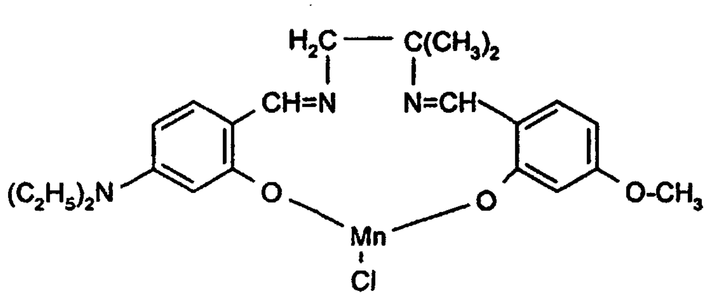

oder

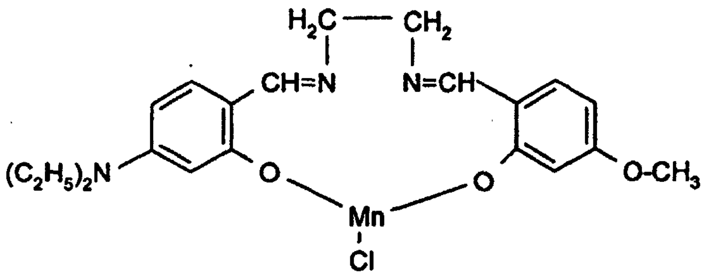

**22.** Waschmittel, enthaltend

I) 5 - 90 % A) eines anionischen Tensids und/oder B) eines nichtionischen Tensids,
II) 5 - 70 % C) einer Buildersubstanz,
III) 0,1 - 30 % D) eines Peroxids und
IV) 0,005 - 2 % E) einer Verbindung der Formel (1) gemäss Anspruch 1, wobei die Prozentangaben jeweils Gewichtsprozente, bezogen auf das Gesamtgewicht des Waschmittels bedeuten.

**23.** Waschmittel gemäss Anspruch 22, enthaltend

I) 5 - 70 % A) eines anionischen Tensids und/oder B) eines nichtionischen Tensids,
II) 5 - 50 % C) einer Buildersubstanz,
III) 1 - 12 % D) eines Peroxids und
IV) 0,02 - 1 % E) einer Verbindung der vorstehend definierten Formel (1), wobei die Prozentangaben jeweils Gewichtsprozente, bezogen auf das Gesamtgewicht des Waschmittels bedeuten.

**24.** Waschmittel, enthaltend

I) 5 - 70 % A) eines anionischen Tensids und/oder B) eines nichtionischen Tensids,
II) 5 - 40 % C) einer Buildersubstanz,
III) 1 - 12 % D) eines Peroxids und
IV) 0,1 - 0,5 % E) einer Verbindung der vorstehend definierten Formel (1), wobei die Prozentangaben jeweils Gewichtsprozente, bezogen auf das Gesamtgewicht des Waschmittels bedeuten.

**25.** Waschmittel gemäss einem der Ansprüche 22 bis 24, enthaltend zusätzlich 0,05 bis 5 Gew. % Polyvinylpyrrolidon.

**26.** Waschmittel gemäss einem der Ansprüche 22 bis 25, enthaltend zusätzlich 0,2 bis 1,7 Gew. % Polyvinylpyrrolidon.

**27.** Waschmittel gemäss einem der Ansprüche 22 bis 26, enthaltend zusätzlich 0,05 bis 5 Gew. % TAED.

**28.** Waschmittel gemäss einem der Ansprüche 22 bis 27, enthaltend zusätzlich 0,2 bis 1,7 Gew. % TAED.

**29.** Verfahren gemäss Anspruch 1, **dadurch gekennzeichnet, dass** man eine Verbindung der Formel (1) zusammen mit einem entsprechenden analogen symmetrischen Mangankomplex einzusetzt, d. h. mit einer Verbindung der Formel (1), worin $(R)_n$ und $(R_1)_m$ identisch sind.

## Claims

**1.** A process for preventing the redeposition of migrating dyes in a wash liquor, which comprises adding to the wash liquor, which comprises a peroxide-containing detergent, from 0.5 to 150 mg per litre of wash liquor of one or more compounds of the formula

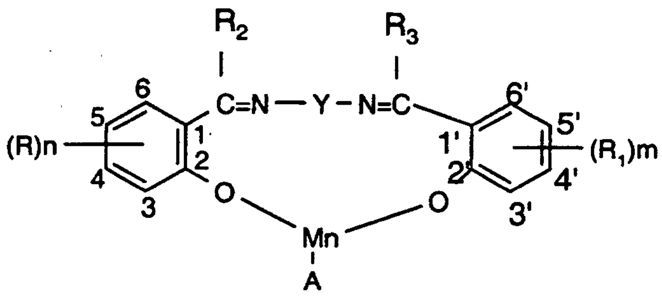

(1)

in which

- n is 0, 1, 2 or 3,
- m is 1, 2 or 3,
- A is an anion;
- Y is a linear or branched alkylene radical of the formula $-[C(R_5)_2]_r-$, where r is an integer from 1 to 8 and the $R_5$ radicals independently of one another are hydrogen or $C_1$-$C_4$alkyl;
  -CX=CX- , in which X is cyano, linear or branched $C_1$-$C_8$alkyl or di(linear or branched $C_1$-$C_8$alkyl)-amino, $-(CH_2)_q-NR_4-(CH_2)_q-$, in which $R_4$ is hydrogen or linear or branched $C_1$-$C_4$alkyl and q is 1, 2, 3 or 4; or
  a 1,2-cyclohexylene radical of the formula:

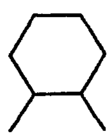

or

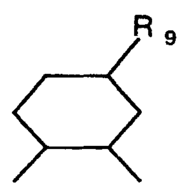

or a 1,2-aryl radical of the formula

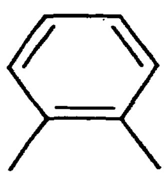

or

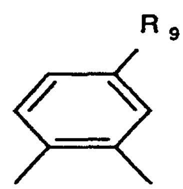

in which $R_9$ is $SO_3H$, $CH_2OH$ or $CH_2NH_2$,

R and $R_1$ independently of one another are cyano, halogen, $OR_5$ or $COOR_5$, in which $R_5$ is hydrogen or linear or branched $C_1$-$C_4$alkyl, or are nitro, linear or branched $C_1$-$C_8$alkyl, linear or branched, partially fluorinated or perfluorinated $C_1$-$C_8$alkyl, $NHR_6$ or $NR_6R_7$ in which $R_6$ and $R_7$ are identical or different and are each linear or branched $C_1$-$C_{12}$alkyl or in which $R_6$ and $R_7$, together with the nitrogen atom connecting them, form a 5-, 6- or 7-membered ring which may include further heteroatoms, or are linear or branched $C_1$-$C_8$alkyl-$R_8$, in which $R_8$ is a radical $OR_5$, $COOR_5$ or $NR_6R_7$ with the above definitions or is $NH_2$, or are $-N^{\oplus}R_4R_6R_7$, in which $R_4$, $R_6$ and $R_7$ are as defined above,

$R_2$ and $R_3$ independently of one another are hydrogen, linear or branched $C_1$-$C_4$ alkyl or unsubstituted aryl, or aryl substituted by cyano, halogen, $OR_5$ or $COOR_5$, in which $R_5$ is hydrogen or linear or branched $C_1$-$C_4$alkyl, or by nitro, linear or branched $C_1$-$C_8$alkyl, $NHR_6$ or $NR_6R_7$ in which $R_6$ and $R_7$ are identical or different and are as defined above, or by linear or branched $C_1$-$C_8$alkyl-$R_8$, in which $R_8$ is a radical $OR_5$, $COOR_5$ or $NR_6R_7$ with the above definitions or is $NH_2$, or by $-N^{\oplus}R_4R_6R_7$, in which. $R_4$, $R_6$ and $R_7$ are as defined above,

with the proviso that R and $R_1$ do not have the same definition if n and m are identical.

**2.** A process according to claim 1, wherein from 1.5 to 75 mg per litre of wash liquor of one or more compounds of the formula (1) are added.

**3.** A process according to claim 2, wherein from 7.5 to 40 mg per litre of wash liquor of one or more compounds of the formula (1) are added.

**4.** A process according to one of claims 1 to 3, wherein the anion A is halide, perchlorate, sulfate, nitrate, hydroxide, $BF_4^-$, $PF_6^-$, carboxylate, triflate or tosylate.

**5.** A process according to claim 4, wherein the anion A is chloride or acetate.

**6.** A process according to one of claims 1 to 5, wherein Y is a radical of the formula $(CH_2)_r$-, where r is an integer from 1 to 8, or of the formula $-C(R_5)_2-(CH_2)_p-C(R_5)_2-$, in which p is a number from 0 to 6 and $R_5$ is hydrogen or $C_1$-$C_4$alkyl.

**7.** A process according to claim 6, wherein Y is a radical of the formula $-(CH_2)_r-$, where r is an integer from 1 to 4, or of the formula $-(CR_5)_2-(CR_5)_2-$, in which $R_5$ independently at each occurrence is hydrogen or methyl.

**8.** A process according to one of claims 1 to 7, wherein halogen is chlorine, bromine or fluorine.

**9.** A process according to one of claims 1 to 8, wherein halogen is chlorine.

**10.** A process according to one of claims 1 to 9, wherein n and/or m is 1 and R and/or $R_1$ are as defined with the exception of nitro and $COOR_5$ and are located in position 4 of the respective benzene ring.

**11.** A process according to one of claims 1 to 10, wherein n and/or m is 1, R and/or $R_1$ are nitro or $COOR_5$ and are located in position 4 of the respective benzene ring, and $R_5$ is hydrogen or linear or branched $C_1$-$C_4$alkyl.

**12.** A process according to one of claims 1 to 11, wherein n and/or m is 2 and R and/or $R_1$ are as defined with the exception of nitro and $COOR_5$ and are located in positions 4 and 6 of the respective benzene ring.

**13.** A process according to one of claims 1 to 12, wherein n and/or m is 2 and R and/or $R_1$ are nitro or $COOR_5$ and are located in positions 3 and 5 of the respective benzene ring.

**14.** A process according to one of claims 1 to 13, wherein R and $R_1$ are nitro, $OR_5$, $COOR_5$ or $N(R_5)_2$, where $R_5$ is hydrogen or $C_1$-$C_4$alkyl.

**15.** A process according to one of claims 1 to 14, wherein R and $R_1$ are nitro, $OR_5$, $COOR_5$ or $N(R_5)_2$, where $R_5$ is hydrogen, methyl or ethyl.

**16.** A compound of the formula

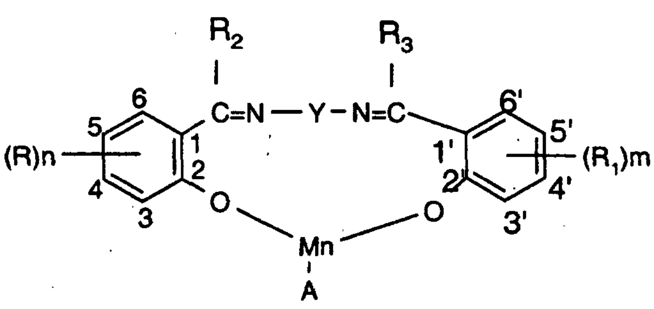

(1a)

in which

- n is 0, 1, 2 or 3,
- m is 1, 2 or 3,
- A is an anion;
- Y is a linear or branched alkylene radical of the formula $-[C(R_5)_2]_r-$, where r is an integer from 1 to 8 and the $R_5$ radicals independently of one another are hydrogen or $C_1$-$C_4$alkyl;

-CX=CX- , in which X is cyano, linear or branched $C_1$-$C_8$alkyl or di(linear or branched $C_1$-$C_8$alkyl)-amino, $-(CH_2)_q-NR_4-(CH_2)_q-$, in which $R_4$ is hydrogen or linear or branched $C_1$-$C_4$alkyl and q is 1, 2, 3 or 4; or a 1,2-cyclohexylene radical of the formula:

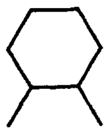

or

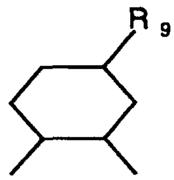

or a 1,2-aryl radical of the formula

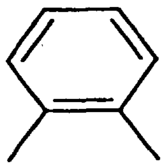

or

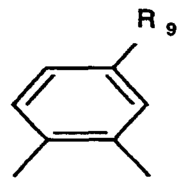

in which $R_9$ is $SO_3H$, $CH_2OH$ or $CH_2NH_2$,
R and $R_1$ independently of one another are cyano, halogen, $OR_5$ or $COOR_5$, in which $R_5$ is hydrogen or linear or branched $C_1$-$C_4$alkyl, or are nitro, linear or branched $C_1$-$C_8$alkyl, linear or branched, partially fluorinated or perfluorinated $C_1$-$C_8$alkyl, $NHR_6$ or $NR_6R_7$ in which $R_6$ and $R_7$ are identical or different and are each linear or branched $C_1$-$C_{12}$alkyl or in which $R_6$ and $R_7$, together with the nitrogen atom connecting them, form a 5-, 6- or 7-membered ring which may include further heteroatoms, or are linear or branched $C_1$-$C_8$alkyl-$R_8$, in which $R_8$ is a radical $OR_5$, $COOR_5$ or $NR_6R_7$ with the above definitions or is $NH_2$, or are $-N^{\oplus}R_4R_6R_7$, in which $R_4$, $R_6$ and $R_7$ are as defined above,
$R_2$ and $R_3$ independently of one another are hydrogen, linear or branched $C_1$-$C_4$ alkyl or unsubstituted aryl, or aryl substituted by cyano, halogen, $OR_5$ or $COOR_5$, in which $R_5$ is hydrogen or linear or branched $C_1$-$C_4$alkyl, or by nitro, linear or branched $C_1$-$C_8$alkyl, $NHR_6$ or $NR_6R_7$ in which $R_6$ and $R_7$ are identical or different and are each linear or branched $C_1$-$C_{12}$alkyl, or in which $R_6$ and $R_7$, together with the nitrogen atom connecting them, form a 5-, 6- or 7-membered ring which may include further heteroatoms, or by linear or branched $C_1$-$C_8$alkyl-$R_8$, in which $R_8$ is a radical $OR_5$, $COOR_5$ or $NR_6R_7$ with the above definitions or is $NH_2$, or by $-N^{\oplus}R_4R_6R_7$, in which $R_4$, $R_6$ and $R_7$ are as defined above,
with the proviso that R and $R_1$ do not have the same definition if n and m are identical and $R_2$ and $R_3$ are both hydrogen and that, of the radicals $R_2$ and $R_3$, it is not the case that one is hydrogen and the other is phenyl.

**17.** A compound according to Claim 16, wherein

| | |
|---|---|
| n and m | are in each case independently of one another 1, 2 or 3, |
| A | is chloride, acetate, triflate or tosylate, |
| Y | is a radical of the formula $-(CH_2)_r-$, where r is an integer from 1 to 8, or of the formula $-C(R_5)_2-(CH_2)_p-C(R_5)_2-$, in which p is a number from 0 to 6 and $R_5$ is hydrogen or $C_1$-$C_4$alkyl, |
| R and $R_1$ | are nitro, $OR_5$, $COOR_5$ or $N(R_5)_2$ where $R_5$ is hydrogen or $C_1$-$C_4$alkyl. |

**18.** A compound according to claim 17, wherein n and m are in each case independently of one another 1, 2 or 3,

| | |
|---|---|
| A | is chloride, acetate, triflate or tosylate, |
| Y | is a radical of the formula $-(CH_2)_r-$, where r is an integer from 1 to 8, or of the formula $-C(R_5)_2-(CH_2)_p-C(R_5)_2-$, in which p is a number from 0 to 6 and $R_5$ is hydrogen or $C_1$-$C_4$alkyl, |
| R and $R_1$ | are nitro, $OR_5$, $COOR_5$ or $N(R_5)_2$ where $R_5$ is hydrogen, methyl or ethyl. |

**19.** A compound of the formula

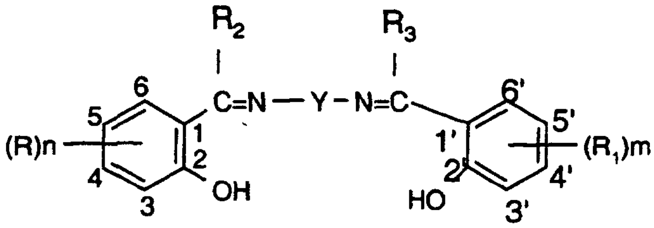

(2)

in which R, $R_1$, $R_2$, $R_3$, Y, n and m are as defined under the formula (1a).

**20.** A process for preparing a compound of the formula (2) according to claim 19, which comprises reacting a diamine of the formula $H_2N$-Y-$NH_2$ first with an aldehyde or ketone of the formula

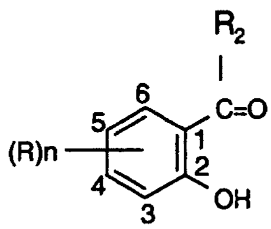

(3)

and then with an aldehyde or ketone of the formula

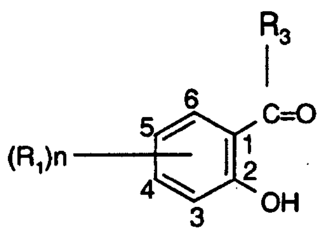

(4)

where R, $R_1$, $R_2$, $R_3$, n and m are as defined under the formula (1).

**21.** A compound of the formula

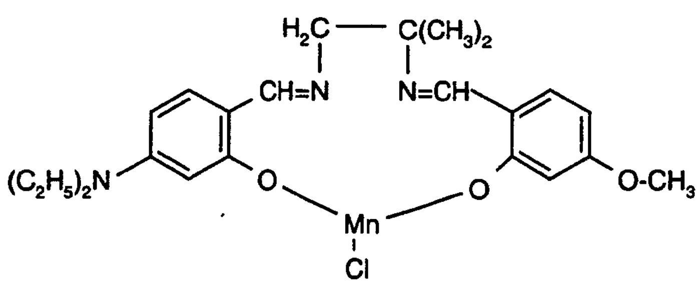

or

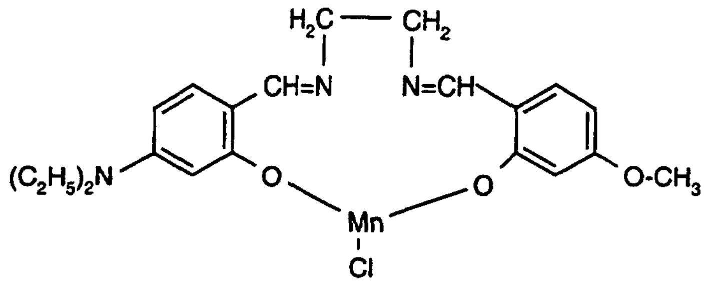

**22.** A detergent comprising

I) 5 - 90% A) of an anionic surfactant and/or B) of a nonionic surfactant,
II) 5 - 70% C) of a builder substance,
III) 0.1 - 30% D) of a peroxide, and
IV) 0.005 - 2% E) of a compound of the formula (1) according to claim 1, the percentages being by weight based on the overall weight of the detergent.

**23.** A detergent according to claim 22 comprising

I) 5 - 70% A) of an anionic surfactant and/or B) of a nonionic surfactant,
II) 5 - 50% C) of a builder substance,
III) 1 - 12% D) of a peroxide, and
IV) 0.02 - 1% E) of a compound of the formula (1) defined above, the percentages being by weight based on the overall weight of the detergent.

**24.** A detergent comprising

I) 5 - 70% A) of an anionic surfactant and/or B) of a nonionic surfactant,
II) 5 - 40% C) of a builder substance,
III) 1 - 12% D) of a peroxide, and
IV) 0.1 - 0.5% E) of a compound of the formula (a) defined above, the percentages being by weight based on the overall weight of the detergent.

**25.** A detergent according to one of claims 22 to 24 additionally comprising from 0.05 to 5% by weight of polyvinylpyrrolidone.

**26.** A detergent according to one of claims 22 to 25 additionally comprising from 0.2 to 1.7% by weight of polyvinylpyrrolidone.

**27.** A detergent according to one of claims 22 to 26 additionally comprising from 0.05 to 5% by weight of TAED.

**28.** A detergent according to one of claims 22 to 27 additionally comprising from 0.2 to 1.7% by weight of TAED.

**29.** A process according to claim 1, wherein a compound of the formula (1) is employed together with a corresponding analogous symmetrical manganese complex, i.e. with a compound of the formula (1) in which $(R)_n$ and $(R_1)_m$ are identical.

**Revendications**

**1.** Procédé pour empêcher la réabsorption de colorants migrants dans un bain détergent, **caractérisé en ce qu'**on ajoute au bain détergent, qui renferme un détergent peroxydé, de 0,5 à 150 mg par litre de bain détergent d'un ou plusieurs composés de formule

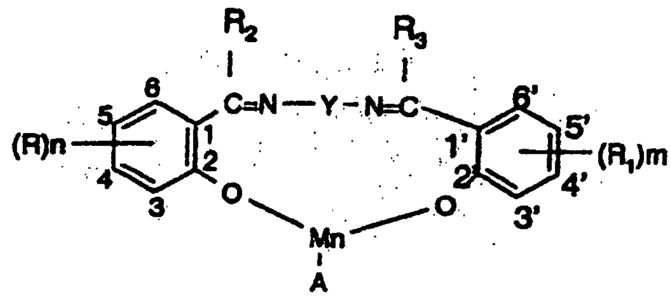

(1)

où

n vaut 0, 1, 2 ou 3,
m vaut 1, 2 ou 3,
A représente un anion ;
Y représente un reste alkylène linéaire ou ramifié de formule $-[C(R_5)_2]_r-$, r est un entier de 1 à 8 et les restes $R_5$ représentent, indépendamment les uns des autres, un atome d'hydrogène ou un groupe alkyle en $C_1$-$C_4$ ;
-CX=CX-, où X représente un groupe cyano, un groupe alkyle en $C_1$-$C_8$ linéaire ou ramifié ou un groupe di (alkyl en $C_1$-$C_8$ linéaire ou ramifié)-amino,
$-(CH_2)_q-NR_4-(CH_2)_q-$, où $R_4$ représente un atome d'hydrogène ou un groupe alkyle en $C_1$-$C_4$ linéaire ou ramifié et
q vaut 1, 2, 3 ou 4 ; ou
un reste 1,2-cyclohexylène de formule

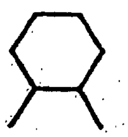

ou

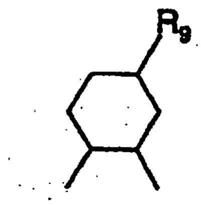

ou un reste 1,2-aryle de formule

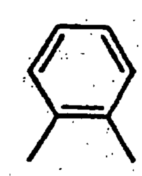

ou

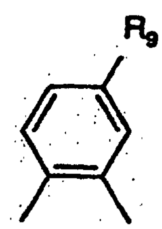

où $R_9$ représente des groupes $SO_3H$, $CH_2OH$ ou $CH_2NH_2$,

R et $R_1$ représentent indépendamment l'un de l'autre des groupes cyano, halogène, $OR_5$ ou $COOR_5$, où $R_5$ représente un atome d'hydrogène ou un groupe alkyle en $C_1$-$C_4$ linéaire ou ramifié, nitro, alkyle en $C_1$-$C_8$ linéaire ou ramifié, alkyle en $C_1$-$C_8$ linéaire ou ramifié partiellement fluoré ou perfluoré, $NHR_6$ ou $NR_6R_7$, où $R_6$ et $R_7$ sont identiques ou différents et représentent chacun un groupe alkyle en $C_1$-$C_{12}$ linéaire ou ramifié, ou dans lesquels $R_6$ et $R_7$ forment conjointement avec l'atome de N qui les relie, un cycle de 5, 6 ou 7 chaînons, qui peut présenter d'autres hétéroatomes, ou un groupe (alkyl en $C_1$-$C_8$)-$R_8$ linéaire ou ramifié, où $R_8$ représente un reste $OR_5$, $COOR_5$ ou $NR_6R_7$ possédant les significations. précitées ou représente $NH_2$, ou $-N^{\oplus}R_4R_6R_7$, où $R_4$, $R_6$ et $R_7$ possèdent les significations données auparavant,

$R_2$ et $R_3$ représentent indépendamment l'un de l'autre un atome d'hydrogène, un groupe alkyle en $C_1$-$C_4$ linéaire ou ramifié ou aryle ou aryle qui est substitué par des substituants cyano, halogène, $OR_5$ ou $COOR_5$ où $R_5$ représente un atome d'hydrogène, un groupe alkyle en $C_1$-$C_4$ linéaire ou ramifié, nitro, alkyle en $C_1$-$C_8$ linéaire ou ramifié, $NHR_6$ ou $NR_6R_7$, où $R_6$ et $R_7$ sont identiques ou différents et possèdent les significations données auparavant, ou un groupe (alkyle en $C_1$-$C_8$)-$R_8$ linéaire ou ramifié où $R_8$ représente un reste $OR_5$, $COOR_5$ ou $NR_6R_7$ possédant les significations précitées ou représente $NH_2$, ou $-N^{\oplus}R_4R_6R_7$, où $R_4$, $R_6$ et $R_7$ possèdent les significations données auparavant,

à condition que R et $R_1$ ne possèdent pas la même signification dans le cas où n et m sont identiques.

**2.** Procédé selon la revendication 1, **caractérisé en ce qu'**on ajoute de 1,5 à 75 mg d'un ou plusieurs composés de formule (1) par litre de bain détergent.

**3.** Procédé selon la revendication 2, **caractérisé en ce qu'**on ajoute de 7,5 à 40 mg par litre de bain détergent d'un où plusieurs composés de formule (1).

**4.** Procédé selon l'une des revendications 1 à 3, **caractérisé en ce** l'anion A représente halogénure, perchlorate, sulfate, nitrate, hydroxyde, $BF_4^-$, $PF_6^-$, carboxylate, triflate ou tosylate.

**5.** Procédé selon la revendication 4, **caractérisé en ce que** l'anion A est chlorure ou acétate.

**6.** Procédé selon l'une des revendications 1 à 5, **caractérisé en ce que** Y représente un reste de formule $-(CH_2)_r-$, r est un entier de 1 à 8 ou de formule $-C(R_5)_2-(CH_2)_p-C(R_5)_2$, où p est un nombre de 0 à 6 et $R_5$ représente un atome d'hydrogène ou un groupe alkyle en $C_1$-$C_4$ linéaire ou ramifié.

**7.** Procédé selon la revendication 6, **caractérisé en ce que** Y représente un reste de formule $-(CH_2)_r-$, où r est un entier de 1 à 4, ou de formule $-(CR_5)_2-(CR_5)_2$, où $R_5$ représente indépendamment un atome d'hydrogène ou un groupe méthyle.

**8.** Procédé selon l'une des revendications 1 à 7, **caractérisé en ce que** l'halogène représente un atome de chlore, de brome ou de fluor.

**9.** Procédé selon l'une des revendications 1 à 8, **caractérisé en ce que** l'halogène représente un atome de chlore.

**10.** Procédé selon l'une des revendications 1 à 9, **caractérisé en ce que** n et/ou m valent 1, R et/ou $R_1$ possèdent l'une des significations données ci-dessus, à l'exception des groupes nitro et $COOR_5$ et se trouvent en position 4 du cycle benzène concerné.

**11.** Procédé selon l'une des revendications 1 à 10, **caractérisé en ce que** n et/ou m valent 1, R et/ou $R_1$ représentent des groupes nitro ou $COOR_5$ et se trouvent en position 4 du cycle benzène concerné, $R_1$ représente un atome d'hydrogène ou un groupe alkyle en $C_1$-$C_4$ linéaire ou ramifié.

**12.** Procédé selon l'une des revendications 1 à 11, **caractérisé en ce que** n et/ou m valent 2, R et/ou $R_1$ possèdent l'une des significations données ci-dessus, à l'exception des groupes nitro et $COOR_5$ et se trouvent en position 4,6 du cycle benzène concerné.

**13.** Procédé selon l'une des revendications 1 à 12, **caractérisé en ce que** n et/ou m valent 2, R et/ou $R_1$ représentent des groupes nitro ou $COOR_5$ et se trouvent en position 3,5 du cycle benzène concerné.

**14.** Procédé selon l'une des revendications 1 à 13, **caractérisé en ce que** R et $R_1$ représentent des groupes nitro, $OR_5$, $COOR_5$ ou $N(R_5)_2$, $R_5$ représentant un atome d'hydrogène ou un groupe alkyle en $C_1$-$C_4$.

**15.** Procédé selon l'une des revendications 1 à 14, **caractérisé en ce que** R et $R_1$ représentent des groupes nitro, $OR_5$, $COOR_5$ ou $N(R_5)_2$, $R_5$ représentant un atome d'hydrogène, un groupe méthyle ou éthyle.

**16.** Composés de formule

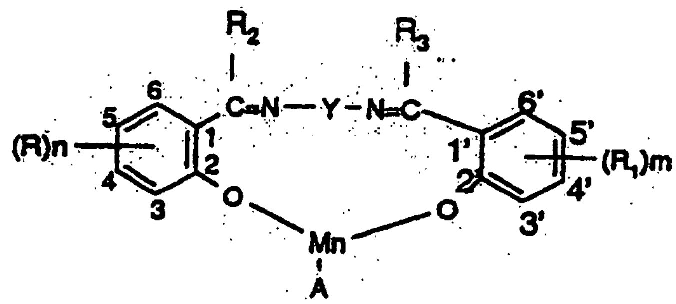

(1a)

où

n vaut 0, 1, 2 ou 3,
m vaut 1, 2 ou 3,
A représente un anion ;
Y représente un reste alkylène linéaire ou ramifié de formule $-[C(R_5)_2]_r-$, r étant un entier de 1 à 8 et les restes $R_5$ représentent, indépendamment les uns des autres, un atome d'hydrogène ou un groupe alkyle en $C_1$-$C_4$ ;
-CX=CX-, où X représente un groupe cyano, un groupe alkyle en $C_1$-$C_8$ linéaire ou ramifié ou un groupe di (alkyl en $C_1$-$C_8$ linéaire ou ramifié)-amino,
$-(CH_2)_q-NR_4-(CH_2)_q-$, où $R_4$ représente un atome d'hydrogène ou un groupe alkyle en $C_1$-$C_4$ linéaire ou ramifié et.
q vaut 1, 2, 3 ou 4 ; ou
un reste 1,2-cyclohexylène de formule

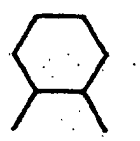

ou

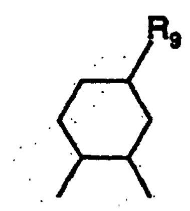

ou un reste 1,2-aryle de formule

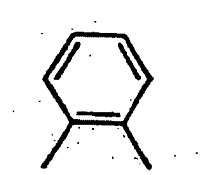

ou

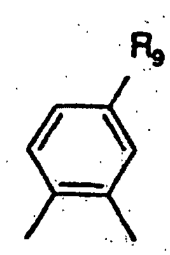

où $R_9$ représente des groupes $SO_3H$, $CH_2OH$ ou $CH_2NH_2$,

R et $R_1$ représentent indépendamment l'un de l'autre des groupes cyano, halogène, $OR_5$ ou $COOR_5$, $R_5$ représente un atome d'hydrogène ou un groupe alkyle en $C_1$-$C_4$ linéaire ou ramifié, nitro, alkyle en $C_1$-$C_8$ linéaire ou ramifié, alkyle en $C_1$-$C_8$ linéaire ou ramifié partiellement fluoré ou perfluoré, $NHR_6$ ou $NR_6R_7$, où $R_6$ et $R_7$ sont identiques ou différents et représentent chacun un groupe alkyle en $C_1$-$C_{12}$ linéaire ou ramifié, ou dans lesquels $R_6$ et $R_7$ forment conjointement avec l'atome de N qui les relie un cycle de 5, 6 ou 7 chaînons, qui peut présenter d'autres hétéroatomes, ou un groupe (alkyl en

$C_1$-$C_8$)-$R_8$ linéaire ou ramifié, où $R_8$ représente un reste $OR_5$, $COOR_5$ ou $NR_6R_7$ possédant les significations précitées ou représente $NH_2$, ou -$N^{\oplus}R_4R_6R_7$, où $R_4$, $R_6$ et $R_7$ possèdent les significations données auparavant,

$R_2$ et $R_3$ représentent indépendamment l'un de l'autre un atome d'hydrogène, un groupe alkyle en $C_1$-$C_4$ linéaire ou ramifié ou aryle non substitué ou aryle qui est substitué par des substituants cyano, halogène, $OR_5$ ou $COOR_5$ où $R_5$ représente un atome d'hydrogène, un groupe alkyle en $C_1$-$C_4$ linéaire ou ramifié, nitro, alkyle en $C_1$-$C_8$ linéaire ou ramifié, $NHR_6$ ou $NR_6R_7$, où $R_6$ et $R_7$ sont identiques ou différents et représentent chacun un groupe alkyle en $C_1$-$C_{12}$ linéaire ou ramifié, ou dans lesquels $R_6$ et $R_7$ forment conjointement avec l'atome d'azote qui les relie un cycle de 5, 6 ou 7 chaînons, qui peut présenter d'autres hétéroatomes, ou représentent un groupe (alkyl en $C_1$-$C_8$)-$R_8$, où $R_8$ représente un reste $OR_5$, $COOR_5$ ou $NR_6R_7$ avec les significations données ci-dessus ou un groupe $NH_2$, ou -$N^{\oplus}R_4R_6R_7$, où $R_4$, $R_6$ et $R_7$ possèdent les significations. données auparavant,

à condition que R et $R_1$ ne possèdent pas la même signification dans le cas où n et m sont identiques et $R_2$ et $R_3$ les deux représentent un atome d'hydrogène et que parmi les restes $R_2$ et $R_3$ l'un ne représente pas un atome d'hydrogène et l'autre un groupe phényle.

**17.** Composés selon la revendication 16, **caractérisés en ce que**

n et m valent chacun indépendamment l'un de l'autre 1, 2 ou 3,
A représente chlorure, acétate, triflate ou tosylate,
Y représente un reste de formule $-(CH_2)_r-$, r est un entier de 1 à 8 ou le reste de formule $-C(R_5)_2-(CH_2)_p-C(R_5)_2$, p est un nombre de 0 à 6 et $R_5$ représente un atome d'hydrogène ou un groupe alkyle en $C_1$-$C_4$,
R et $R_1$ représentent des groupes nitro, $OR_5$, $COOR_5$ ou $N(R_5)_2$, où $R_5$ un atome d'hydrogène ou un groupe alkyle en $C_1$-$C_4$.

**18.** Composés selon la revendication 17, **caractérisés en ce que**

n et m valent chacun indépendamment l'un de l'autre 1, 2 ou 3,
A représente chlorure, acétate, triflate ou tosylate,
Y représente un reste de formule $-(CH_2)_r-$, r étant un entier de 1 à 8, ou de formule $-C(R_5)_2-(CH_2)_p-C(R_5)_2$, où p est un nombre de 0 à 6 et $R_5$ représentant un atome d'hydrogène ou un groupe alkyle en $C_1$-$C_4$,
R et $R_1$ représentent des groupes nitro, $OR_5$, $COOR_5$ ou $N(R_5)_2$, où $R_5$ représente un atome d'hydrogène, un groupe méthyle ou éthyle.

**19.** Composés de formule

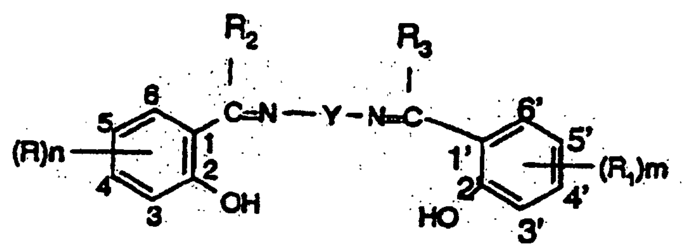

(2)

où R, $R_1$, $R_2$, $R_3$, Y, n et m possèdent la signification donnée à la formule (1a).

**20.** Procédé pour la préparation de composés de formule (2) selon la revendication 19, **caractérisé en ce qu'**on fait réagir une diamine de formule $H_2N$-Y-$NH_2$ d'abord sur un aldéhyde ou une cétone de formule

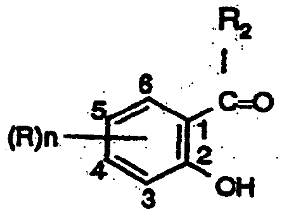

(3)

et ensuite sur un aldéhyde ou une cétone de formule

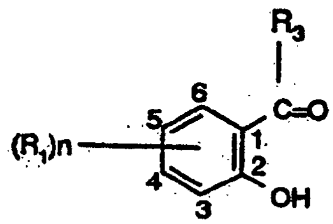

(4)

R, $R_1$, $R_2$, $R_3$, n et m possèdant la signification donnée à la formule (1).

**21.** Composés de formule

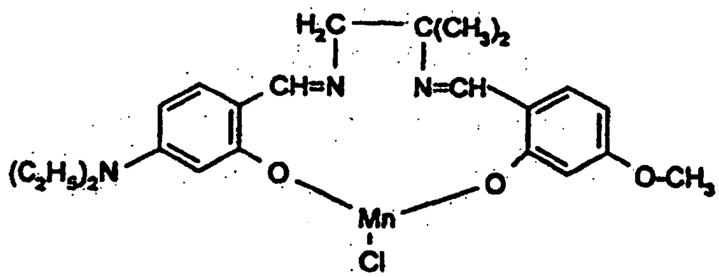

ou

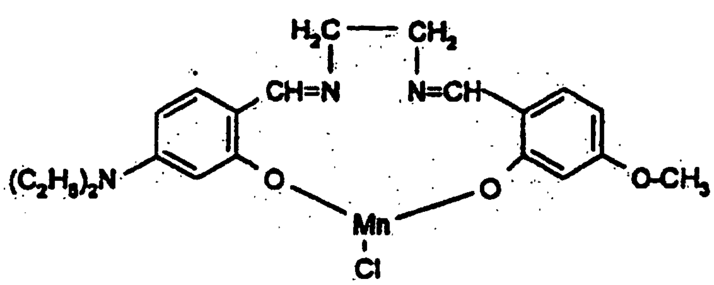

**22.** Détergent renfermant

I) de 5 à 90 % A) d'un agent de surface anionique et/où B) d'un agent de surface non ionique,
II) de 5 à 70 % d'un adjuvant,
III) de 0,1 à 30 % D) d'un peroxyde et
IV) de 0,005 à 2 % E) d'un composé de formule (1) selon la revendication 1,

les indications en pourcentages signifient chaque fois des pour cents en poids par rapport au poids total du détergent.

**23.** Détergent renfermant

I) de 5 à 70 % A) d'un agent de surface anionique et/ou B) d'un agent de surface non ionique,
II) de 5 à 50 % C) d'un adjuvant de détergent,
III) de 1 à 12 % D). d'un peroxyde et
IV) de 0,02 à 1 % E) d'un composé de formule (1) définie auparavant,

les indications en pourcentages signifient chaque fois des pour cents en poids par rapport au poids total du détergent.

**24.** Détergent renfermant

I) de 5 à 70 % A) d'un agent de surface anionique et/ou B) d'un agent de surface non ionique,
II) de 5 à 40 % C) d'un adjuvant,
III) de 1 à 12 % D) d'un peroxyde et
IV) de 0,1 à 0, 5 % E) d'un composé de formule (1) définie auparavant,

les indications en pourcentages signifient chaque fois des pour. cents en poids par rapport au poids total du détergent.

**25.** Détergent selon l'une des revendications 22 à 24, renfermant en plus de 0,05 à 5 % en poids de polyvinylpyrrolidone.

**26.** Détergent selon l'une des revendications, 22 à 25, renfermant en plus de 0,2 à 1,7 % en poids de polyvinylpyrrolidone.

**27.** Détergent selon une ou plusieurs des revendications 22 à 26, renfermant en plus de 0,05 à 5 % en poids de TAED.

**28.** Détergent selon l'une des revendications 22 à 27, renfermant en plus de 0,2 à 1,7 % en poids de TAED.

**29.** Procédé selon la revendication 1, **caractérisé en ce qu'**on utilise un composé de formule (1) conjointement avec un complexe de manganèse symétrique analogue correspondant, c'est-à-dire avec un composé de formule (1), où $(R)_n$ et $(R_1)_m$ sont identiques.